# EUROPEAN PATENT SPECIFICATION

(11) **EP 3 082 845 B1**
(45) Date of publication and mention of the grant of the patent: **17.02.2021**
(21) Application number: 14872066.7
(22) Date of filing: 17.12.2014
(51) Int. Cl.: A61K 38/17, A61K 31/216, A61K 31/46, A61K 31/496, A61K 31/5513, A61P 25/02

(54) **METHODS AND COMPOSITIONS FOR TREATMENT OF PERIPHERAL NEUROPATHIES**
VERFAHREN UND ZUSAMMENSETZUNGEN ZUR BEHANDLUNG VON PERIPHEREN NEUROPATHIEN
PROCÉDÉS ET COMPOSITIONS POUR LE TRAITEMENT DE NEUROPATHIES PÉRIPHÉRIQUES

(30) Priority: 20.12.2013 US 201361919301 P
(43) Date of publication of application: 26.10.2016
(73) Proprietor: University Of Manitoba, Winnipeg, Manitoba R3T 5V4 (CA)
(72) Inventor: FERNYHOUGH, Paul, Winnipeg, Manitoba R3T 5V4 (CA); CALCUTT, Nigel A., Encinitas CA 92024 (US)
(74) Representative: HGF
(86) International application number: PCT/CA2014/051227
(87) International publication number: WO 2015/089664

(56) References cited:
- WO-A1-2012/055018
- WO-A1-2012/055018
- WO-A1-2012/112933
- PLOEG VAN DER R ET AL: "DEVELOPMENT OF THERAPEUTIC CANDIDATES TO TREAT DIABETIC SENSORY NEUROPATHY", 2ND ANNUAL CANADIAN NEUROSCIENCE MEETING. HILTON BONAVENTURE HOTEL. MONTREAL, CANADA. MAY 25TH - 28TH 2008, , 25 May 2008 (2008-05-25), pages 1-2, XP002726599, Retrieved from the Internet: URL:http://www.can-acn.org/meeting2008/abs tracts/257.htm [retrieved on 2014-02-10]
- SMITH D R ET AL: "Selective antagonism of the muscarinic acetylcholine receptor type 1 activates AMPK, augments neurite outgrowth and reverses diabetic sensory neuropathy", SOCIETY FOR NEUROSCIENCE ABSTRACT VIEWER AND ITINERARY PLANNER, vol. 43, November 2013 (2013-11), XP002769911, & 43RD ANNUAL MEETING OF THE SOCIETY-FOR-NEUROSCIENCE; SAN DIEGO, CA, USA; NOVEMBER 09 -13, 2013
- MIZUNAGA M ET AL: "INTRAVESICAL INSTILLATION OF OXYBUTYNIN HYDROCHLORIDE THERAPY FOR PATIENTS WITH A NEUROPATHIC BLADDER", PARAPLEGIA, INTERNATIONAL MEDICAL SOCIETY OF PARAPLEGIA, EDINBURGH, GB, vol. 32, 1 January 1994 (1994-01-01), pages 25-29, XP000944002, ISSN: 0031-1758
- PATEL ET AL: "Can Oxybutynin Cause Peripheral Neuropathy?", JOURNAL OF UROL, LIPPINCOTT WILLIAMS & WILKINS, BALTIMORE, MD, US, vol. 168, no. 2, 1 August 2002 (2002-08-01), page 646, XP005540157, ISSN: 0022-5347, DOI: 10.1016/S0022-5347(05)64704-0
- STEPANOVIC PETROVIC R M ET AL: "The treatment of delayed polyneuropathy induced by diisopropylfluorophosphate in hens", DIE PHARMAZIE: AN INTERNATIONAL JOURNAL OF PHARMACEUTICAL SCIE, GOVI VERLAG PHARMAZEUTISCHER VERLAG GMBH, DE, vol. 55, no. 6, 1 June 2000 (2000-06-01), pages 454-455, XP009194321, ISSN: 0031-7144
- HAYASHIDA K I ET AL: "Inhibitory M2 muscarinic receptors are upregulated in both axotomized and intact small diameter dorsal root ganglion cells after peripheral nerve injury", NEUROSCIENCE, NEW YORK, NY, US, vol. 140, no. 1, 1 January 2006 (2006-01-01), pages 259-268, XP024986742, ISSN: 0306-4522, DOI: 10.1016/J.NEUROSCIENCE.2006.02.013 [retrieved on 2006-01-01]
- VAN DER PLOEG ET AL.: 'Development of Therapeutic Candidates to Treat Diabetic Sensory Neuropathy' 2ND ANNUAL CANADIAN NEUROSCIENCE MEETING May 2008, XP002726599 Retrieved from the Internet: <URL:http://www.can-acn.org/meeting2008/abs tracts/257.htm> [retrieved on 2015-02-23]
- DE LAET ET AL.: 'Systemic oxybutynin decreases afferent activity of the pelvic nerve of the rat: new insights into the working mechanism of antimuscarinics' NEUROUROLOGY AND URODYNAMICS vol. 25, no. 2, 2006, pages 156 - 161, XP055352268

## Description

### FIELD OF THE INVENTION

The invention relates to compositions for therapeutic treatment of peripheral neuropathy disorders, to the use of the compositions, and to methods for use of the compositions.

### BACKGROUND

Peripheral neuropathy is a clinical problem in persons affected with diabetes or treated with chemotherapeutic agents. Peripheral neuropathies can be also caused by infections such as HIV and leprosy. The clinical symptoms may include development of upper back and/or abdominal pain (i.e., thoracoabdominal neuropathy), loss of control of eye movements (i.e., third-nerve palsy), and progressive loss of function of the nerves comprising the peripheral nervous system (e.g., polyneuropathy, mononeuropathy, mononeuritis simplex, autonomic neuropathy). Peripheral neuropathies include the following: neuropathy associated with diabetes mellitus (diabetic neuropathy), HIV-associated neuropathy; nutritional deficiency-associated neuropathy; cranial nerve palsies; drug-induced neuropathy; industrial neuropathy; lymphomatous neuropathy; myelomatous neuropathy; multi-focal motor neuropathy; immune-mediated disorders, chronic idiopathic sensory neuropathy; carcinomatous neuropathy; acute pain autonomic neuropathy; alcoholic neuropathy; compressive neuropathy; vasculitic/ischaemic neuropathy; mono- and polyneuropathies. There are a range of genetically inherited peripheral neuropathies exemplified by Charcot-Marie-Tooth (CMT) disease and all its forms, and Friedreich's ataxia. Other peripheral neuropathies may arise from Raynaud's Phenomenon (including CREST syndrome), leprosy and autoimmune diseases such as erythromatosis and rheumatoid diseases.

Therapeutic compositions may cause occurrences of peripheral neuropathies, particularly those used for the treatment of neoplastic disease. In certain cases, peripheral neuropathy is a major complication of cancer treatment and is the main factor limiting the dosage of chemotherapeutic agents that can be administered to a patient (Cavaletti et al. 2013, The chemotherapy-induced peripheral neuropathy outcome measures standardization study: from consensus to the first validity and reliability findings. Ann. Oncol. 24:454-462). Chemotherapy-induced peripheral neuropathy (CIPN) often occurs during treatment of various cancers and other disorders with a variety of agents including taxanes (i.e. paclitaxel/taxol), platinum-based drugs (i.e. cisplatin), vinka alkaloids (i.e. vincristine) proteasome inhibitors (e.g., Bortezomib) and agents that alter cancer cell metabolism (e.g. dichloroacetate). CIPN can limit dose and duration of treatment, thereby reducing efficacy of the chemotherapeutic regime. Up to 40% of cancer patients treated with chemotherapy describe some form of CIPN, with sensory neuropathy frequently being dominant. Symptoms vary from tingling and numbness indicative of sensory loss to aspects of painful neuropathy such as allodynia and spontaneous shooting pains that usually start in the hands and feet before moving proximally. Conduction velocity of large sensory and motor fibers may also be impaired upon electrophysiological testing. CIPN is dose dependent and lowering the dose of the chemotherapeutic or completely withdrawing treatment can reduce or eliminate symptoms over a period that may be from days to months.

Paclitaxel is a microtubule-stabilizing drug that impedes cell division and this is the presumed basis of its chemotherapeutic properties. Paclitaxel is commonly used to treat breast, lung and ovarian cancers, but is dose limited by CIPN. The manifestation of CIPN in paclitaxel-intoxicated rodents is protocol dependent, possibly reflecting the dose-related nature of the clinical condition.

Studies of paclitaxel-induced neuropathy in patients and rodents suggest pathogenic mechanisms that include disruption of organelle transport in axons via microtubule reorganization and damage to Schwann cells and satellite cells. There is accumulating evidence that paclitaxel, and other instigators of CIPN share a common pathogenic mechanism involving mitochondrial dysfunction. The resulting energy depletion has the potential to impede high ATP consuming processes, such as actin treadmilling and consequent peripheral terminal plasticity. Retraction and degeneration of the peripheral terminals of sensory C fibers has been noted in paclitaxel-treated animals.

Dichloroacetate (DCA) is an environmental toxin that is also use to treat mitochondrial diseases by virtue of its ability to inhibit pyruvate dehydrogenase (PDH) kinase, which results in increased PDH activity and therefore increased flow of pyruvate into the electron transport chain, with the end result of increasing ATP production. It has been recently recognized that DCA can kill certain cancerous cells by disrupting their inherently anaerobic metabolism. Patients given DCA to treat mitochondrial disease or cancer develop an unwanted side effect of peripheral neuropathy that presents as tingling, loss of sensation and/or pain in the extremities and which can be dose limiting to the point of causing cessation of DCA treatment.

The dominant form of neuropathy in the majority of neuropathic diseases is a distal symmetrical polyneuropathy that initially affects subjects' feet, legs and hands. The primary symptoms include loss of touching and/or feeling sensations and the loss of ability to sense pain-causing stimuli. A sub-group of patients also develop positive symptoms of neuropathic pain such as inappropriate tingling, burning, shooting or aching sensations that may co-exist with other negative symptoms of sensory loss. Such neuropathic pain is commonly referred to as tactile allodynia or mechano-hyperalgesia.

Distal sensory neuropathy is a major component of symmetrical polyneuropathy and can be measured using skin biopsies to determine loss of intraepidermal nerve fibers (IENF) (Kennedy et al., 1996, Quantitation of epidermal nerves in diabetic neuropathy. Neurology 47:1042-48). IENF loss represents retraction of sensory neuron nerve endings from the epidermis with subsequent sensory loss that ultimately contributes to high incidences of ulceration, gangrene and amputation in subjects suffering peripheral neuropathy. Currently, there are no regulatory approved therapies available in North America for degenerative symmetrical polyneuropathy. The current costs to health systems for providing relief of these symptoms are enormous.

Muscarinic acetylcholine receptor antagonists block binding of acetylcholine to muscarinic receptors (G-protein coupled receptors, i.e., GPCRs, with sub types of M1, M2, M3, M4 and M5). Antimuscarinic drugs were found to treat both the negative (nerve conduction slowing and sensory loss) and positive (allodynia/hyperalgesia) symptoms of diabetic symmetrical polyneuropathy, not only ameliorating but also reversing nerve damage. Several muscarinic acetylcholine receptor antagonists promote sensory neuron growth in vitro and significantly prevented and/or reversed loss of intraepidermal and corneal nerve fibers, thermal hypoalgesia, large fiber conduction slowing and tactile allodynia, symptoms commonly associated with peripheral neuropathies. Pirenzepine, a selective M1 receptor antagonist that acts as a competitive inhibitor via interaction with the orthosteric site of the receptor, proved particularly efficacious. The most specific antagonist of the M1 receptor is muscarinic toxin 7 (MT7), a 64-66 amino acid protein, derived from the African green mamba snake (*Dendroaspis angusticeps*)*.* This protein binds to an allosteric site on the M1 receptor at low nanomolar concentrations and shows 10,000-fold selectivity for M1 receptors over M2-M5 (Max et al., 1993, Stable allosteric binding of ml-toxin to M1 muscarinic receptors. Mol. Pharmacol. 44:1171-5). A less selective compound is the competitive antagonist oxybutynin (i.e., 4-diethylaminobut-2-ynyl-2-cyclohexyl-2-hydroxy-2-phenyl-ethanoate) is commonly available in oral compositions and/or transdermal compositions exemplified by OXYTROL® (OXYTROL is a registered trademark of Actavis Inc., Parsippany, NJ, USA), DITROPAN®, DITROPAN XL® (DITROPAN and DITROPAN XL are registered trademarks of Alza Corp., Mountain View, CA, USA), GELNIQUE® (GELINQUE is a registered trademark of Actavis Inc., Parsippany, NJ, USA), Lyrinel XL, Ditrospam, and Urotrol. Oxybutynin is commonly prescribed to relieve urinary and bladder disorders, including but not limited to urinary incontinence, overactive bladder, enuresis, neuropathic bladder, nephrotuberculosis, neurogenic bladder, and detrusor overactivity. Oxybutynin is also effective for treating and/or managing postoperative pain related to an indwelling bladder catheter, hyperhidrosis, and refractory hot flashes in cancer patients. Oxybutynin acts as a selective competitive antagonist of acetylcholine at muscarinic receptors (M1, M2 and M3), resulting in relaxation of smooth muscle.

### SUMMARY

The invention is set forth in claims 1 to 12. It is to be understood that the following description is exemplary and explanatory and describes the invention only to the extent that it discloses subject matter within the scope of the claims.

The exemplary embodiments of the disclosure pertain to compositions comprising pirenzipine or salt thereof useful for therapy of peripheral neuropathies. Exemplary therapeutic compositions may further comprise one or more of oxybutynin, muscarinic toxin 7 (MT7), muscarinic receptor antagonists, and the like. Alternatively, exemplary therapeutic compositions may further comprise one or more of a salt of oxybutynin, a salt of MT7, a salt of a muscarinic receptor antagonist, and the like. Alternatively, exemplary therapeutic compositions may further comprise one or more of a derivative of oxybutynin, a derivative of MT7, a derivative of a muscarinic receptor antagonist, and the like. The compositions are suitable for treating both the negative symptoms of peripheral neuropathy exemplified by nerve conduction slowing and by sensory loss, and the positive symptoms of peripheral neuropathy exemplified by tactile allodynia and by mechanohyperalgesia.

Other exemplary methods pertain to methods for manufacturing the topical compositions of the present disclosure. Other exemplary methods pertain to methods for manufacturing the oral compositions of the present disclosure.

### DESCRIPTION OF THE DRAWINGS

The present disclosure will be described in conjunction with reference to the following drawings in which:
Fig. 1 is a chart showing dose-dependent effects of selective receptor antagonists on neurite outgrowth from dissociated sensory neurons that were derived from adult Sprague Dawley rats;
Figs 2(A) and 2(B) are charts showing the effects of pirenzepine (PZ) on nerve conduction velocity (NCV) in streptozotocin (STZ)-induced diabetic rats;
Figs. 3(A), 3(B), 3(C), 3(D) show the effects of treatment time with pirenzepine on activation of AMP-activated protein kinase (AMPK) in control rats (Figs, 3(A), 3(B)) and diabetic rats (Figs. 3(C), 3(D));
Figs. 4(A)-4(D) are micrographs of GFP fluorescence images of adult sensory neuron cultures derived from STZ-induced diabetic rats showing blockade by dominant negative mutants of AMPK of pirenzepine-induced neurite outgrowth;
Figs. 5(A), 5(B) are charts showing the total neurite outgrowth in the cultures shown in Figs. 4(A)-4(D);
Fig. 6 is a chart showing 1 µM pirenzepine enhanced transcriptional activity of peroxisome proliferator-activated receptor-γ coactivator-1α (PGC-1α);
Figs. 7(A), 7(B) are charts showing that sensory neuron respiration (related to mitochondrial-dependent oxidative phosphorylation), was augmented in neuronal cultures derived from (A) M1 receptor knockout mice (M1R KO), of (B) adult cultures from STZ-induced diabetic rats treated with 1 µM VU0255035;
Figs. 8(A), 8(B) are charts showing the coupling efficiency in neuronal cultures derived from M1R KO mice (A) and in STZ-induced diabetic rats treated with VU0255035 (B), Figs. 8(C), 8(D) are charts showing the respiratory control ratio in neuronal cultures derived from M1R KO mice (C) and in STZ-induced diabetic rats treated with VU0255035 (D), and 8(E), 8(F) are charts showing the spare respiratory capacity in neuronal cultures derived from M1R KO mice (E) and in STZ-induced diabetic rats treated with VU0255035 (F);
Fig. 9(A) is a micrograph of sensory neurons derived from normal adult rats, while Fig. 9(B) is a micrograph of the effects of muscarinic toxin 7 (MT7) on neurite outgrowth in these sensory neurons;
Fig. 10 is a chart showing that MT7 dose-dependently elevated total neurite outgrowth in sensory neurons derived from normal adult rats;
Figs. 11(A), 11(B) are charts showing the effects of MT7 (A) and co-treatment with MT7 and compound C (the inhibitor of AMPK), (B) on transcriptional activity of PGC-1α in cultured adult sensory neurons derived from adult STZ-induced diabetic rats;
Fig. 12 is a chart showing the inhibition of pirenzepine-induced neurite outgrowth in cultured adult sensory neurons derived from adult rats by the Ca²⁺/calmodulin-dependent kinase kinase (CaMKK) inhibitor, STO-609;
Figs. 13(A), 13(B) show the inhibition of pirenzepine-induced activation of AMPK in cultured adult sensory neurons from normal adult rats by the CaMKK inhibitor, STO-609, while Figs. 13(C), 13(D) show the inhibition of MT7-induced activation of AMPK in cultured sensory neurons from normal adult rats by the CaMKK inhibitor, STO-609;
Fig. 14 is a chart showing that oxybutynin elevated neurite outgrowth in cultured adult sensory neurons;
Fig. 15 is a chart showing the ability of oxybutynin to reverse loss of paw thermal sensitivity in a mouse model of type 2 diabetes (control is "C57"; diabetic is "db/db"; oxybutynin treated is "db/db + oxy");
Fig. 16(A) is a chart showing the effects of topical oxybutynin on intraepidermal nerve fiber (IENF) density in genetically diabetic db/db mice, while Fig. 16(B) is a chart showing the effects of topical oxybutynin on nerve fibers within the cornea of diabetic db/db mice;
Fig. 17 is a chart showing effects of systemic oxybutynin on development of thermal hypoalgesia in STZ-induced diabetic Swiss Webster mice;
Fig. 18(A) is a chart showing that pirenzepine enhanced neurite outgrowth in the presence of the chemotherapy agent paclitaxel in cultures of adult sensory neurons, while Fig. 18(B) is a chart showing that pirenzepine enhanced neurite outgrowth in the presence of the chemotherapy agent oxaliplatin in cultures of adult sensory neurons;
Fig. 19 is a chart showing paw thermal response latency (left panel) and 50% tactile response threshold (right panel) in mice treated with paclitaxel (taxol) ± pirenzepine (Pz);
Figs. 20(A), 20(B), 2(C) are charts showing the effects of pirenzepine (Pz) on paw thermal hypoalgesia (A), depletion of paw skin IENF (B) and on onset of MNCV slowing in mice treated with dichloroacetate (DCA)(C);
Fig. 21 is a chart showing the effects of atroprine delivered topically to the hind paws or eyes of STZ-induced diabetic mice on MNCV;
Fig. 22 is a chart showing the effects of atroprine delivered topically to the hind paw or eyes of STZ-induced diabetic mice on paw thermal latency;
Fig. 23 is a chart showing the effects of 11 days of topical application of MT7 to the eye of control mice on nerve fiber occupancy in the sub-basal nerve plexus (SBNP 1-5) and stroma (Stroma 1-10) of the cornea; and
Fig. 24 is a chart showing protective effect of pirenzepine and MT7 on neurite outgrowth in an in vitro model of HIV-induced neuropathy.

### DETAILED DESCRIPTION

Unless defined otherwise, all technical and scientific terms used herein have the meanings that would be commonly understood by one of skill in the art in the context of the present specification. Although any methods and materials similar or equivalent to those described herein can also be used in the practice or testing of the present disclosure, the preferred methods and materials are now described. All publications mentioned herein disclose and describe the methods and/or materials in connection with which the publications are cited.

It must be noted that as used herein and in the appended claims, the singular forms "a", "an", and "the" include plural references unless the context clearly dictates otherwise. Thus, for example, reference to "an agent" includes a plurality of such agents and reference to "the subject" includes reference to one or more subjects and equivalents thereof known to those skilled in the art, and so forth.

"Optional" or "optionally" or "alternatively" means that the subsequently described event, circumstance, or material may or may not occur or be present, and that the description includes instances where the event, circumstance, or material occurs or is present and instances where it does not occur or is not present.

Where a range of values is provided, it is understood that each intervening value, to the tenth of the unit of the lower limit unless the context clearly dictates otherwise, between the upper and lower limit of that range and any other stated or intervening value in that stated range, is encompassed within the disclosure. The upper and lower limits of these smaller ranges may independently be included in the smaller ranges, and are also, encompassed within the disclosure, subject to any specifically excluded limit in the stated range. Where the stated range includes one or both of the limits, ranges excluding either or both of those included limits are also included in the disclosure.

"Inhibit", "inhibiting", and "inhibition" mean to decrease an activity, response, condition, disease, or other biological parameter. This can include but is not limited to the complete ablation of the activity, response, condition, or disease. This may also include, for example, a 10% reduction in the activity, response, condition, or disease as compared to the native or control level. Thus, the reduction can be a 10%, 20%, 30%, 40%, 50%, 60%, 70%, 80%, 90%; 100%, or any amount of reduction in between the specifically recited percentages, as compared to native or control levels.

"Promote", "promotion", and "promoting" refer to an increase in an activity, response, condition, disease, or other biological parameter. This can include but is not limited to the initiation of the activity, response, condition, or disease. This may also include, for example, a 10% increase in the activity, response, condition, or disease as compared to the native or control level. Thus, the increase in an activity, response, condition, disease, or other biological parameter can be a 10%, 20%, 30%, 40%, 50%, 60%, 70%, 80%, 90%, 100%, or more, including any amount of increase in between the specifically recited percentages, as compared to native or control levels.

As used herein, the term "subject" means any target of administration. The subject can be a vertebrate, for example, a mammal. Thus, the subject can be a human. The term does not denote a particular age or sex. Thus, adult, juvenile, and newborn subjects, whether male or female, are intended to be covered. A patient refers to a subject afflicted with a disease or disorder. The term "patient" includes human and veterinary subjects.

As used herein, the terms "treatment", "treating", and the like, refer to obtaining a desired pharmacologic and/or physiologic effect. The effect may be prophylactic in terms of completely or partially preventing a disease or symptom thereof and/or may be therapeutic in terms of a partial or complete cure for a disease and/or adverse effect attributable to the disease. "Treatment", as used herein, covers any treatment of a disease in a mammal, particularly in a human, and includes: (a) preventing the disease from occurring in a subject which may be predisposed to the disease but has not yet been diagnosed as having it; (b) inhibiting the disease, i.e., arresting its development; and (c) relieving the disease, i.e., causing regression of the disease.

The term "therapeutically effective" means that the amount of the composition used is of sufficient quantity to ameliorate one or more causes or symptoms of a disease or disorder. Such amelioration only requires a reduction or alteration, not necessarily elimination. The "therapeutically effective amount" will vary depending on the compound, the disease and its severity and the age, weight, etc., of the subject to be treated.

As used herein, "pharmaceutical composition" includes any composition for: (i) topical administration, or (ii) transdermal administration or (iii) parenteral administration, or (iv) oral administration, of oxybutynin, pirenzepine, muscarinic toxin 7 (referred to herein after as "MT7"), muscarinic receptor antagonist, and the like to a subject in need of therapy for peripheral neuropathy. Pharmaceutical compositions may include carriers, thickeners, diluents, buffers, preservatives, surface active agents and the like in addition to oxybutynin, pirenzepine, MT7, muscarinic receptor antagonist, and the like. Pharmaceutical compositions may also include one or more active ingredients such as antimicrobial agents, antiinflammatory agents, anaesthetics, analgesics, and the like.

Muscarinic acetylcholine receptor antagonists reverse loss of intraepidermal nerve fibers and thermal hypoalgesia in peripheral neuropathies. Muscarinic acetylcholine receptor antagonists are agents that reduce the activities and/or function of muscarinic acetylcholine receptors that are found in the plasma membranes of neurons and other cells. Muscarinic acetylcholine receptors are GPCRs that are stimulated by acetylcholine released from several cell types including sensory neurons, keratinocytes and postganglionic fibers in the parasympathetic nervous system, and function as signaling molecules that initiate signal cascades within cells in their immediate regions. Well-known muscarinic acetylcholine receptor antagonists useful for treatment of maladies such as central nervous system malfunctioning, pulmonary diseases, and gastric ailments are exemplified by atropine, scopolamine, pirenzepine, telenzepine, hyoscine, hyoscyamine, ipratropium, tropicamide, cyclopentolate, glycopyrrolate, 4-diphenylacetoxy-1,1-dimethylpiperidinium, quinidine, orphenadrine, oxybutynin, oxyphenonium, emepronium, procyclidine, propantheline, 4-fluorhexahydrosiladifenidol, octylonium, quinuclidinyl benzilate, tolterodine, benactyzine, fesoterodine (fumarate), trospium, solifenacin, gallamine, bipreiden, dicyclomine, benztropine, dexetimide, hexahydrosiladifenidol, among others.

Furthermore, in addition to pirenzepine, there are a number of selective antagonists for the type 1 muscarinic receptor (MIR) and other muscarinic receptor subtypes that are anticipated to exhibit beneficial activities similar to those demonstrated by pirenzepine on neuronal neurite outgrowth. Some of these compounds have much superior M1R selective activity. For example, telenzepine, an analog of pirenzepine with an altered tricyclic structure but an unmodified piperazine side chain, is 4 to 10 times more potent than pirenzepine. VU0255035, a thiadiazole derivative and is 75 times more selective to M1R relative to M2, M3, M4 and M5 receptors. Among the new generation of M1R antagonists, promising centrally active M1R antagonists include PD150714, and 77-LH-28-1 and spirotramine. Listings of muscarinic acetylcholine receptor antagonists suitable for incorporation into therapeutic compositions for peripheral neuropathy include: muscarinic toxin 7 (MT7) green mamba venom; tricyclic benzodiazepinone derivatives (such as pirenzepine, telenzepine); 1,4-disubstituted tetrahydropyridine carboxylic acids exemplified by PD150714; trihexyphenidyl analogs exemplified by trihexyphenidyl and p-fluorotrihexyphenidy; thiadiazole sulfonamide derivatives exemplified by VU0255035; hexocyclium and sila-hexocyclium exemplified by O-methoxy-sila-hexocyclium; polymethylene tetraamine or spiro-4-damp(4-diphenyl-acetoxy-n-methylpiperidine exemplified by spirotramine; n-(4-(4-ethylpiperazin-1-yl) phenyl amide analogues; McN-A-343 analogues; alkoxy-oxadiazolyltetrahydropyridines exemplified by MB-OXTP; caramiphen, aprophen and related derivatives exemplified by nitrocaramiphen and aprophen; (-)-S-ET126; N-desmethylclozapine; MDL74019DG; glycopyrronium bromide; and dicyclomine. Also included are M1R mixed antagonists, i.e. compounds that show antagonist effects at more than one subtype of muscarinic receptor, including M1, such as rispenzepine, R-procyclidine, and DAU 5750. Other muscarinic antagonists include nuvenzepine, 4-fluorohexahydrosiladifenidol, 4-diphenylacetoxy-N-methyl-piperidine methiodide, tolterodine, PD102807, oxybutynin, iptratropium bromide, and the like.

Variations to the structures of the above descriptions of muscarinic antagonists could make these compounds more selective M1R antagonists, or alternatively, be varied for stability, safety or efficacy. Accordingly, generic formulae can be designed that encompass the structural features for the antagonists, including those that are M1R selective and M1R-non-selective compounds. Suitable formulae are exemplified by Formula I and Formula II. 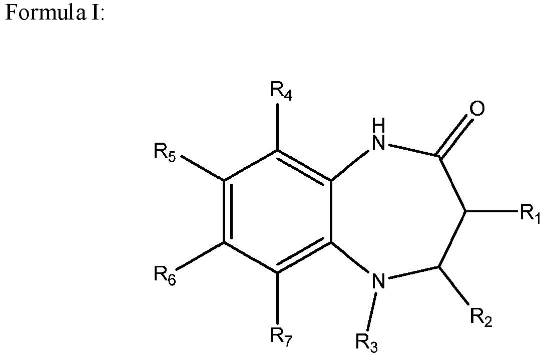
R1 may be one of a 5-membered unsaturated ring, a 6-membered unsaturated ring, or a hetero atom-containing ring;
R2 may be one of a 5-membered unsaturated ring, a 6-membered unsaturated ring, or a hetero atom-containing ring;
R3 may be one of a H-piperidinyl group, a 2-piperidinyl group, a 3-piperidinyl, a 4-piperidinyl group, a 2-piperazinyl group, or a 3-piperazinyl group, linked via a methyl group or an ethyl group or a propyl group or a butyl group. The piperidinyl groups or piperazinyl groups may additionally be linked to methyl, trifluoromethyl or ethyl moieties.
R4 may be a hydrogen ion or a chloride ion.
R5 may be a hydrogen ion or a chloride ion.
R6 may be a hydrogen ion or a chloride ion.
R7 may be a hydrogen ion or a chloride ion. 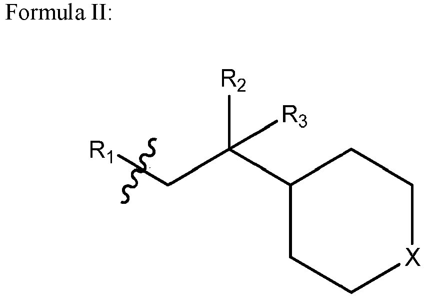
X may be a methyl group or a "NR" group i.e. a primary amine group, a secondary amine group, or a tertiary amine group.
R1 may be 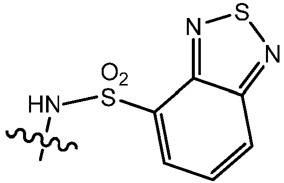
wherein " " indicates the point of connection of R1 with the upper structure in Formula II.
R2 may be a hydroxyl ion or a hydrogen ion or a ketone.
R3 may be a hydroxyl ion or a hydrogen ion or a ketone.

The term "unit dosage form" as used herein, refers to physically discrete units suitable as unitary dosages for human and animal subjects, each unit containing a predetermined quantity of pirenzepine, calculated in an amount sufficient to produce the desired effect in association with a pharmaceutically acceptable diluent, carrier or vehicle.

The term "carrier" means a compound, composition, substance, or structure that, when in combination with pirenzepine, aids or facilitates preparation, storage, administration, delivery, effectiveness, selectivity, or any other feature of pirenzepine, for its intended use or purpose. For example, a carrier can be selected to minimize any degradation of the pirenzepine, and to minimize any adverse side effects in the subject.

Suitable pharmaceutically acceptable carriers include essentially chemically inert and nontoxic pharmaceutical compositions that do not interfere with the effectiveness and/or safety of the primary biological activity of the pharmaceutical composition. Suitable carriers and their formulations are described in Remington (1995, The Science and Practice of Pharmacy (19th ed.) ed. A. R. Gennaro, Mack Publishing Company, Easton, PA). Typically, an appropriate amount of a pharmaceutically acceptable salt is used in the formulation to render the formulation isotonic. Examples of suitable pharmaceutical carriers include, but are not limited to, saline solutions, glycerol solutions, ethanol, N-(1(2,3 -dioleyloxy)propyl)-N,N,N-trimethylammonium chloride (DOTMA), diolesylphosphotidylethanolamine (DOPE), and liposomes. Such pharmaceutical compositions should contain a therapeutically effective amount of the compound, together with a suitable amount of carrier so as to provide the form for proper administration to the subject. The formulation should suit the mode of administration. For example, oral administration requires enteric coatings to protect pirenzepine, from degradation within the gastrointestinal tract. In another example, the pirenzepine, may be administered in a liposomal formulation to facilitate transport throughout a subject's vascular system and effect delivery across cell membranes to intracellular sites.

The term "excipient" herein means any substance, not itself a therapeutic agent, which may be used in a composition for delivery of pirenzepine, to a subject or alternatively combined with pirenzepine, (e.g., to create a pharmaceutical composition) to improve its handling or storage properties or to permit or facilitate formation of a dose unit of the composition (e.g., formation of a topical hydrogel which may then be optionally incorporated into a transdermal patch). Excipients include, by way of illustration and not limitation, binders, disintegrants, taste enhancers, solvents, thickening or gelling agents (and any neutralizing agents, if necessary), penetration enhancers, solubilizing agents, wetting agents, antioxidants, lubricants, emollients, substances added to mask or counteract a disagreeable odor, fragrances or taste, substances added to improve appearance or texture of the composition and substances used to form the pharmaceutical compositions. Any such excipients can be used in any dosage forms according to the present disclosure. The foregoing classes of excipients are not meant to be exhaustive but merely illustrative as a person of ordinary skill in the art would recognize that additional types and combinations of excipients could be used to achieve the desired goals for delivery of pirenzepine.

Peripheral neuropathy is an urgent, unmet clinical need. Nerve degeneration and impaired regeneration are pathological hallmarks of peripheral neuropathy. Diabetic neuropathy most frequently manifests as a distal symmetrical polyneuropathy, afflicting both the somatic and autonomic divisions of the peripheral nervous system. Diabetic neuropathy is often initially described as a small fiber neuropathy and skin biopsies reveal the early retraction of small sensory fibers from the epidermis (Said, 2007, Diabetic neuropathy - a review. Nat. Clin. Pract. Neurol. 3:331-340). Segmental demyelination and axonal degeneration of large fibers are seen in nerve biopsies from patients after many years of diabetes, along with clusters of regenerating small fibers (Kalichman et al., 1998, Reactive, degenerative, and proliferative Schwann cell responses in experimental galactose and human diabetic neuropathy. Acta Neuropath. 95:47-56). The capacity of injured small fibers to regenerate has been shown to be impaired in repeated skin biopsies from diabetic subjects (Polydefkis et al. 2004, The time course of epidermal nerve fibre regeneration: studies in normal controls and in people with diabetes, with and without neuropathy. Brain 127:1606-1615). It appears that peripheral nerves are in a constant dynamic equilibrium between nerve retraction and re-growth and that the diverse metabolic stresses of systemic diabetes enhance retraction and impede regeneration, leading to distal nerve degeneration.

Peripheral neuropathy is generally recognized by patients at onset of sensory symptoms such as pain, dysesthesia's and/or sensory loss in the extremities. Physicians can confirm the diagnosis using simple sensory tests (15 g monofilament and tuning fork) or comprehensive scoring systems that encompass pain scores, sensory and autonomic function tests, and detailed large fiber electrophysiology. Historically, most clinical trials of therapies designed to prevent or alleviate peripheral neuropathy have used large fiber electrophysiology as the primary end point for efficacy, even where therapies have targeted small fiber neuropathy. Trials have occasionally included sural nerve biopsies to evaluate the pathology underlying symptoms but this approach is invasive and not recommended. There is an emerging focus on small fiber pathology to measure peripheral neuropathy and efficacy of interventions, as small sensory fiber terminals can be assessed quantitatively and iteratively in longitudinal studies using minimally invasive skin biopsy or non-invasive corneal confocal microscopy techniques. IENF and corneal nerve depletion, representing mostly small unmyelinated sensory fibers that transduce thermal sensation, correlates well with symptoms such as pain and sensory loss and also with measures of large fiber neuropathy such as conduction slowing. Moreover, rodent models of peripheral neuropathy also develop IENF and corneal nerve depletion. This offers a logical and scientifically consistent pathway for development of therapies that promote nerve regeneration after injury, starting with in vitro studies to assess potential for promoting axonal growth after the trauma of dissecting dorsal root ganglia (DRG), then in vivo studies in rodent models of peripheral neuropathy to assess IENF numbers and function and then clinical studies that measure the same parameter.

The term "neuropathy" includes any pathology or abnormality of neural tissue causing nerve dysfunction. The function of the nerve or nerves that is disrupted may involve the rate of flow of the electrical current through the nerve or may involve ectopic firing (firing in the absence of stimulus) of the nerve, or may involve inappropriate or inadequate firing of the nerve in response to a stimulus. Peripheral neuropathy as used herein is defined as a disorder resulting from damage to peripheral nerves. It may be acquired, caused by diseases of the nerves or as the result of systemic illness.

A number of factors can cause, induce or are associated with peripheral neuropathies, and included within the scope of the disclosure are peripheral neuropathies associated with, diabetes, infections exemplified by HIV, toxic agents, alcoholism, nutritional deficiencies, systemic/metabolic disorders, palsies, auto-immune disorders, inherited or genetic disorders, cancers and tumors, compressive neuropathies; vasculitic/ischaemic neuropathy; mono- and polyneuropathies. In further embodiments, the peripheral neuropathy manifests as a post surgical complication.

Preferably included within the scope of the term neuropathy/neuropathies are neuropathies associated with diseases such as: uremia; childhood cholestatic liver disease; chronic respiratory insufficiency; alcoholic polyneuropathy; multiple organ failure; sepsis; hypoalbuminemia; eosinophilia-myalgia syndrome; hypoglycemia; vitamin or nutritional deficiency (e.g., B-12 deficiency, vitamin A deficiency, vitamin E deficiency, vitamin B1 deficiency); primary biliary cirrhosis; hyperlipidemia; sensory perineuritis; allergic granulomatous angiitis; hypersensitivity angiitis; Bell's Palsy, Wegener's granulomatosis; rheumatoid arthritis; systemic lupus erythematosis; mixed connective tissue disease; scleroderma; systemic vasculitides; acute tunnel syndrome; pandysautonomia; hypothyroidism; chronic obstructive pulmonary disease; acromegaly; malabsorption (sprue, celiac disease); carcinomas (sensory, sensorimotor, late and demyelinating); lymphoma (including Hodgkin's), polycythemia vera; multiple myeloma (lytic type, osteosclerotic, or solitary plasmacytoma); tropical myeloneuropathies; pernicious anemia, Churg-Strauss syndrome; cranial nerve palsies; drug-induced neuropathy; industrial neuropathy; lymphomatous neuropathy; myelomatous neuropathy, chronic idiopathic sensory neuropathy; carcinomatous neuropathy; acute pain autonomic neuropathy; compressive neuropathy; mono- and polyneuropathies; or diabetes.

In preferred embodiments, the peripheral neuropathy is a diabetic neuropathy. It will be clearly understood that the diabetic neuropathy may be diabetic or pre-diabetic associated with Type 1 (insulin-dependent) diabetes, Type 2 (non-insulin-dependent) diabetes, or both.

In other embodiments the peripheral neuropathy is induced by or alternatively, a secondary affect due to a toxic agent such as a drug, industrial chemical or environmental toxin. For example, the peripheral neuropathy can be caused by a chemotherapeutic agent such as paclitaxel (or other taxane derivative), alkaloids such as vincristine or vinblastin, platinum compounds such as cisplatin, carboplatin, oxaliplatin, dichloroacetate, topoisomerase inhibitors, intercalators such as bleomycin, or drugs such as chloramphenicol, colchicine, dapsone, disulfiram, amiodarone, gold, isoniazid, misonidazole, nitrofurantoin, perhexiline, propafenone, pyridoxine, phenytoin, simvastatin, tacrolimus, thalidomide, cyclophosphamide or zalcitabine, an agent used for the treatment of infectious diseases such as streptomycin, didanosine or zalcitabine, or any other chemically toxic agent such as acrylamide, arsenic, carbon disulfide, hexacarbons, lead, mercury, platinum, an organophosphate, thallium, or alcohol.

In another preferred embodiment, the peripheral neuropathy caused by a systemic or metabolic disease is selected from the group consisting of diabetic or prediabetic neuropathy, acquired primary demyelinating neuropathy, distal symmetric sensory polyneuropathy, distal symmetric sensorimotor polyneuropathy, vasculitic neuropathy, infectious neuropathy, idiopathic neuropathy, immune-mediated neuropathy, nutrition-related neuropathy, kidney or liver failure, and paraneoplastic neuropathy.

In other embodiments, the peripheral neuropathy is induced by an infection or infectious disease, such as leprosy, Lyme disease, HIV or acquired immunodeficiency syndrome (AIDS), post-polio syndrome, herpes simplex and herpes zoster (aka shingles); hepatitis B, hepatitis C, HIV, cytomegalovirus, or diphtheria.

In a preferred embodiment, immune-mediated such as acquired primary demyelinating neuropathy includes chronic inflammatory demyelinating polyradiculoneuropathy (CIDP), Guillain-Barre syndrome / acute inflammatory demyelinating polyneuropathy (AIDP), sarcoidosis; vasculitic/ischaemic neuropathy (such as polyarteritis nodosa, rheumatoid arthritis, systemic lupus erythematosus (Lupus) and Sjogren's syndrome, celiac disease (sprue), multi-focal motor neuropathy (MNN), or peripheral neuropathy associated with protein abnormalities (such as monoclonal gammopathy, amyloidosis, cryoglobulinemia, macroglobulinemia, POEMS).

In a preferred embodiment, the compression that causes peripheral neuropathy is selected from the group consisting of carpal tunnel syndrome, ulnar neuropathy at the elbow or wrist, common peroneal nerve at the knee, tibial nerve at the knee, amyloidosis, and sciatic nerve.

Also included within the scope of the term neuropathy/neuropathies are hereditary or genetically acquired neuropathies, including peroneal muscular atrophy (Charcot-Marie-Tooth Disease) hereditary amyloid neuropathies, hereditary sensory neuropathy (type I and type II), porphylias or porphyric neuropathy, hereditary (neuropathy) liability to pressure palsy (HNPP), Fabry's Disease, adrenomyeloneuropathy, Riley-Day Syndrome, Dejerine-Sottas neuropathy (hereditary motor-sensory neuropathy-III), Refsum's disease, Raynaud's disease including CREST syndrome. Krabbe's disease, ataxia- telangiectasia, hereditary tyrosinemia, anaphalipoproteinemia, abetalipoproteinemia, giant axonal neuropathy, metachromatic leukodystrophy, globoid cell leukodystrophy, or Friedrich's ataxia.

The compositions and methods of the disclosure can be also be used to treat or prevent neuropathy related to or induced by the following diseases, trauma, or conditions: general neuropathic conditions, such as peripheral neuropathy, phantom limb pain, reflex-sympathetic dystrophy, causalgia, syringomyelia, and painful scar; specific neuralgias at any location of the body; back pain; diabetic neuropathy; alcoholic neuropathy; metabolic neuropathy; inflammatory neuropathy; chemotherapy-induced neuropathy, herpetic neuralgias; traumatic odontalgia; endodontic odontalgia; thoracic-outlet syndrome; cervical, thoracic, or lumbar radiculopathies with nerve compression; cancer with nerve invasion; traumatic-avulsion injuries; mastectomy, thoracotomy pain; spinal-cord-injury; stroke; abdominal-cutaneous nerve entrapments; tumors of neural tissues; arachnoiditis; stump pain; fibromyalgia; regional sprains or strains; myofascial pain; psoriatic arthropathy; polyarteritis nodosa; osteomyelitis; burns involving nerve damage; AIDS-related pain syndromes; connective tissue disorders, such as systemic lupus erythematosis, systemic sclerosis, polymyositis, and dermatomyositis; and inflammatory conditions, such as acute inflammation (e.g. trauma, surgery and infection) or chronic inflammation (e.g., arthritis and gout).

Peripheral nerves undergo continuous cholinergic constraint. Structurally distinct and selective (e.g. pirenzepine) or specific (e.g. MT7) M1 muscarinic receptor antagonists promote neurite outgrowth in primary cultures of sensory neurons derived from adult rodents. Importantly, neurite outgrowth is also enhanced in cultured sensory neurons derived from mice lacking the M1 receptor compared to those derived from normal mice. Along with preclinical data shown below, this underlies the general hypothesis that peripheral sensory neurons exist under "cholinergic constraint" that prevents excessive growth of peripheral terminals. This cholinergic constraint may use autocrine and/or paracrine secretion mechanisms involving the neurotransmitter acetylcholine (the primary endogenous ligand for muscarinic receptors). Adult rat sensory neurons contain a peripheral form of the acetylcholine (ACh) synthesizing enzyme choline acetyltransferase (pChAT), exhibit ChAT activity and express both the vesicular ACh transporter and the M1 receptor. This explains why M1 receptor antagonists show efficacy in the cell culture systems. A paracrine mechanism may also operate as epidermal keratinocytes and cells of the cornea secrete ACh and communicate via cholinergic receptors.

This concept of endogenous restraint of axon growth has some precedence in studies of the CNS. Moreover, preclinical data in type 1 and type 2 diabetic rodents and rodents with CIPN and DCA-induced neuropathy demonstrates that a therapeutic strategy in which neurons are protected from degeneration and encouraged towards regeneration by targeting the endogenous cholinergic constraint system using muscarinic receptor antagonists is viable. Muscarinic receptor antagonists are not new drugs and a number are in clinical use throughout the world to treat a variety of diseases separate and distinct from diabetic and other peripheral neuropathies.

A common feature that unifies the etiology of nerve degeneration in numerous peripheral nerve diseases involves impaired mitochondrial function. Inability of mitochondria to produce sufficient ATP leads to nerve degeneration and the failure of nerves to regenerate after stress and/or damage (Roy Chowdhury et al., 2013, The role of aberrant mitochondrial bioenergetics in diabetic neuropathy. Neurobiol. Dis. 51: 56-65).

### Diabetic neuropathy:

Sensory neurons in animal models of type 1 and type 2 diabetes exhibit impaired mitochondrial gene expression and function. Studies on neurons derived from diabetic rodents reveals that their mitochondrial membrane potentials are depressed (Huang et al., 2003, Insulin prevents depolarization of the mitochondrial inner membrane in sensory neurons of type 1 diabetic rats in the presence of sustained hyperglycemia. Diabetes 52:2129-36). Analysis of the bioenergetics of sensory neurons from diabetic rodents shows sub-optimal maximal respiration capacity and loss of activity of electron transport complexes (Roy Chowdhury et al., 2012, Impaired AMP-activated protein kinase signaling in dorsal root ganglia neurons is linked to mitochondrial dysfunction and peripheral neuropathy in diabetes. Brain 135:1751-66). Changes in the mitochondrial proteome underlie these alterations and are driven by impaired activation of AMP-activated protein kinase (AMPK) - a master regulator of mitochondrial function and fidelity (Roy Chowdhury et al., 2012; Akude et al., 2011, Diminished superoxide generation is associated with respiratory chain dysfunction and changes in the mitochondrial proteome of sensory neurons from diabetic rats. Diabetes 60:288-97). In diabetic neuropathy, this down-regulation of mitochondrial function in adult sensory neurons is associated with suppression of AMPK and PGC-la activity (Roy Chowdhury et al. 2012, Impaired adenosine monophosphate-activated protein kinase signaling in dorsal root ganglia neurons is linked to mitochondrial dysfunction and peripheral neuropathy in diabetes. Brain 135: 1751-66). The mechanism of M1 receptor mediated inhibition of axonal growth involves the down-regulation of mitochondrial bioenergetics and function. Releasing neurons from this constraint using M1 receptor antagonists leads to activation of AMPK, increased transcriptional activity of PGC-la and associated enhancement of mitochondrial respiration. The M1 receptor modulates this pathway at a proximal level via CaMKKβ (or CaMKK2), a described activator of AMPK. AMPK is a multi-component Ser/Thr kinase activated by binding of AMP upon a rise in the AMP/ATP ratio. Activated AMPK switches on catabolic pathways, primarily through optimization of mitochondrial function, to produce ATP while simultaneously shutting down energy-consuming anabolic processes. AMPK activation increases phosphorylation of the transcription factor PGC-la and AMPK requires PGC-la activity to modulate the expression of several key players in metabolism, including components of the mitochondrial electron transport system. Deacetylation of PGC-la by the cytoplasmic sirtuins, SIRT1 and SIRT2, increases its transcriptional activity. Coupled regulation of PGC-la by AMPK and sirtuins plays a major role in the metabolic adaptations to energy metabolism in different tissues.

The positive effects of pirenzepine on neurite outgrowth, phosphorylation of AMPK and transcriptional activation of PGC-la are replicated by MT7. Oxybutynin also elevates neurite outgrowth in cultured sensory neurons and, like pirenzepine, protects mice with type 1 and type 2 diabetes from development of sensory neuropathy.

### Chemotherapy-induced peripheral neuropathy (CIPN):

Paclitaxel and oxaliplatin induce CIPN through disruption of mitochondrial function that drives distal axonal loss. (Bennett et al., 2011, Terminal arbor degeneration--a novel lesion produced by the antineoplastic agent paclitaxel. Eur. J. Neurosci. 33:1667-76; Xiao et al., 2011, Mitochondrial abnormality in sensory, but not motor, axons in paclitaxel-evoked painful peripheral neuropathy in the rat. Neuroscience 199:461-9; Zheng et al., 2011, Functional deficits in peripheral nerve mitochondria in rats with paclitaxel- and oxaliplatin-evoked painful peripheral neuropathy. Exp. Neurol. 232:154-61). In rodent models of CIPN anlaysis of mitochondrial electron transport activity revealed deficits in capacity and was linked to dying back of axons in the skin (Zheng et al., 2011). Pirenzepine protected cultured neurons from degeneration induced by the CIPN-inducing agents paclitaxel and oxaliplatin. In vivo studies with mice treated with paclitaxel revealed pirenzepine protected from development of thermal and tactile allodynia. Pirenzepine also protected DCA-treated mice from development of thermal hypoalgesia, IENF loss and MNCV slowing.

### Charcot-Marie-Tooth (CMT):

In Charcot-Marie-Tooth disease type 2 (CMT2) a distal dying-back axonal degeneration is predominant and in 19% to 33% of cases, has been linked to mutations in the GTPase and mitochondrial fusion protein, mitofusin 2 (MFN2) (Zuchner, et al., 2004, Mutations in the mitochondrial GTPase mitofusin 2 cause Charcot-Marie-Tooth neuropathy type 2A. Nat. Genet. 36:449-51). Mutant MFN2 over-expressed in sensory neurons also resulted in a distal dying-back neuropathy in mouse models that was characterized by compromised axonal trafficking of mitochondria (Baloh et al., 2007, Altered axonal mitochondrial transport in the pathogenesis of Charcot-Marie-Tooth disease from mitofusin 2 mutations. J. Neurosci. 27:422-30). The bioenergetics properties of mitochondria were not significantly altered and the role of MFN2 in mediating transport was deemed to be discrete from regulation of mitochondrial fusion (Baloh et al., 2007; Misko et al., 2010, Mitofusin 2 is necessary for transport of axonal mitochondria and interacts with the Miro/Milton complex. J. Neurosci. 30:4232-40).

### HIV-induced neuropathy:

Post-mortem peripheral nerve samples from patients with HIV-induced neuropathy reveal increased mutation in mitochondrial DNA (mutation mtDNA⁴⁹⁷⁷) that was associated with deficits in mitochondrial protein expression (Lehmann et al., 2011, Mitochondrial dysfunction in distal axons contributes to human immunodeficiency virus sensory neuropathy. Ann Neurol. 69, 100-10). These insufficiencies were more pronounced at a distal level compared with more proximal nerve segments. Simian immunodeficiency virus (SIV) infected macaques exhibited similar abnormalities, with abnormal markers of mitochondrial function and an elevation in mitochondrial-dependent reactive oxygen species (ROS) production (Lehmann et al., 2011). Studies with cultured human DRG treated with supernatants from HIV-infected macrophages also reveal mitochondrial dysfunction (manifesting as membrane depolarization) with signs of oxidative stress in perikarya but not in axons (Hahn et al., 2008, Differential effects of HIV infected macrophages on dorsal root ganglia neurons and axons. Exp. Neurol. 210:30-40). The alterations in mitochondrial phenotype (e.g., inner membrane depolarization and depressed expression of proteins), mirror the defects seen in diabetic neuropathy.

### Friedreich ataxia:

This is an autosomal recessive neurodegenerative disease induced by a GAA repeat expansion in intron 1 of the frataxin gene. The resulting diminished expression is linked with a dying-back neuropathy impacting sensory neurons and spinocerebellar and corticospinal motor tracts (Puccio et al., 2002, Friedreich ataxia: a paradigm for mitochondrial diseases. Curr. Opin. Genet. Dev. 12:272-77). Frataxin is an iron chaperone required for formation of iron-sulfur (Fe-S) clusters, but its loss is associated with both diminished activity of Fe-S-containing enzymes (important for optimal mitochondrial respiratory chain function) and with deficient defenses against oxidative stress (Bencze et al., 2006, The structure and function of frataxin. Crit. Rev. Biochem. Mol. Biol. 41 :269-91). In mouse models of the disease, mitochondrial dysfunction occurs in the absence of any enhancement of oxidative stress (Seznec et al., 2005, Friedreich ataxia: the oxidative stress paradox. Hum. Mol. Genet. 14:463-74). Some clarity on the etiology of this disease has come from work in *Drosophila* where mitochondrial inner membrane depolarization preceded impaired mitochondrial trafficking and distal loss of fibers in the absence of oxidate stress (Shidara & Hollenbeck, 2010, Defects in mitochondrial axonal transport and membrane potential without increased reactive oxygen species production in a Drosophila model of Friedreich ataxia. J. Neurosci. 30:11369-78). There are distinct parallels with mitochondrial dysfunction observed in diabetic neuropathy. Inner membrane depolarization in sensory neurons is identified early in the disease in animal models and respiratory chain dysfunction is observed in the absence of any attendant elevation in ROS production (Akude et al., 2011; Huang et al, 2003; Roy Chowdhury et al, 2012).

Antimuscarinic drugs can protect peripheral nerves from degeneration in a number of common diseases by manipulating cellular AMPK levels and therefore mitochondrial function. Data is presented below showing that these drugs can repair nerve damage in diabetes, CIPN, HIV and DCA toxicity. These are diseases caused by very different initial stressors that share a common mitochondrial component in the etiology or progression of disease. Importantly, the ability of these drugs to repair mitochondrial fidelity and drive nerve regeneration occurs independent from any role for muscarinic signaling in the etiology of the disease. The inhibition of the cholinergic constraint pathway by these drugs enables nerve regeneration and repair to be enhanced across a broad range of diseases.

This disclosure provides compositions comprising agents that include, but are not limited to, oxybutynin, pirenzepine, MT7, muscarinic receptor antagonist, and the like to treat peripheral neuropathy induced by diabetes, chemotherapeutic agents including DCA, HIV, and genetic diseases exemplified by Charcot-Marie-Tooth disease. The agents can be formulated as pharmaceutical compositions for various routes of delivery.

In another embodiment, agents exemplified by oxybutynin, pirenzepine, MT7, other muscarinic receptor antagonists and the like are used to restore reduced AMPK activity and PGC-1α transcriptional activity.

Each of the foregoing agents (e.g., oxybutynin, pirenzepine, MT7, muscarinic receptor antagonist, and the like), can be formulated in a pharmaceutical composition for various routes of administration.

In one embodiment, the pharmaceutical compositions disclosed herein comprise an agent as described above (e.g., oxybutynin, pirenzepine, MT7, muscarinic receptor antagonist, and the like), in a total amount by weight of the composition of about 0.1% to about 95%. For example, the amount of oxybutynin, pirenzepine, MT7, muscarinic receptor antagonist, and the like, by weight of the pharmaceutical composition may be about 0.1%, about 0.2%, about 0.3%, about 0.4%, about 0.5%, about 0.6%, about 0.7%, about 0.8%, about 0.9%, about 1 %, about 1.1%, about 1.2%, about 1.3%, about 1.4%, about 1.5%, about 1.6%, about 1.7%, about 1.8%, about 1.9%, about 2%, about 2.1%>, about 2.2%, about 2.3%, about 2.4%, about 2.5%, about 2.6%, about 2.7%, about 2.8%, about 2.9%, about 3%, about 3.1%, about 3.2%, about 3.3%, about 3.4%, about 3.5%, about 3.6%, about 3.7%, about 3.8%, about 3.9%, about 4%, about 4.1%, about 4.2%, about 4.3%, about 4.4%, about 4.5%, about 4.6%, about 4.7%, about 4.8%. about 4.9%, about 5%, about 5.1 %, about 5.2%, about 5.3%, about 5.4%., about 5.5%, about 5.6%, about 5.7%, about 5.8%, about 5.9%, about 6%, about 6.1%, about 6.2%, about 6.3%, about 6.4%, about 6.5%, about 6.6%, about 6.7%, about 6.8%, about 6.9%, about 7%, about 7.1%, about 7.2%, about 7.3%, about 7.4%, about 7.5%, about 7.6%, about 7.7%, about 7.8%, about 7.9%, about 8%, about 8.1%, about 8.2%, about 8.3%, about 8.4%, about 8.5%, about 8.6%, about 8.7%, about 8.8%, about 8.9%, about 9%, about 9.1%, about 9.2%, about 9.3%, about 9.4%, about 9.5%, about 9.6%, about 9.7%, about 9.8%, about 9.9%, about 10%, about 1 1%, about 12%, about 13%, about 14%, about 15%, about 16%, about 17%, about 18%, about 19%, about 20%, about 25%, about 30%, about 35%, about 40%, about 45%, about 50%, about 55%, about 60%, about 65%, about 70%, about 75%, about 80%, about 85%, about 90% or about 95%.

The pharmaceutical compositions of the disclosure comprising agent(s) may be formulated for topical administration or alternatively, for transdermal administration.

A pharmaceutical composition for topical administration may be provided as, for example, ointments, creams, suspensions, lotions, powders, solutions, pastes, gels, hydrogels, sprays, aerosols, dressings, or oils. When formulated in an ointment, the active ingredient may be employed with either a paraffmic or a water-miscible ointment base. Alternatively, the active ingredient may be formulated in a cream with an oil-in-water base or a water-in-oil base. Other formulations the compositions can be incorporated into include oils, suppositories, foams, liniments, aerosols, buccals, and sublingual tablets or topical devices for absorption through the skin or mucous membranes.

Ointments and creams may, for example, be formulated with an aqueous or oily base with the addition of suitable thickening and/or gelling agents. Lotions may be formulated with an aqueous or oily base and will in general also contain one or more emulsifying agents, stabilizing agents, dispersing agents, suspending agents, thickening agents, or coloring agents. Liquid sprays are conveniently delivered from pressurized packs, for example, via a specially shaped closure. Oil-In-Water emulsions can also be utilized in the compositions, patches, bandages and articles. These systems are semisolid emulsions, micro-emulsions, or foam emulsion systems. Usually such a system has a "creamy white" appearance. The oleaginous phase may contain, but is not limited to, long-chain alcohols (cetyl, stearyl), long-chain esters (myristates, palmitates, stearates), long-chain acids (palmitic, stearic), vegetable and animal oils and assorted waxes. These can be made with anionic, cationic, nonionic or amphoteric surfactants, or with combinations especially of the nonionic surfactants. A typical invention gel base, provided herein for exemplary purposes only, can contain lecithin, isopropyl palmitate, poloxamer 407, and water. Topical carriers with different viscosities and hand-feel are known to the art. The above active ingredients can be dispersed within the pharmaceutically acceptable carrier in therapeutically effective amounts to treat neuropathies, and the other maladies described above.

A pharmaceutical composition for transdermal administration may be provided as, for example, a hydrogel comprising agents as described herein incorporated into an adhesive patch composition intended to remain in intimate contact with a subject's epidermis for a prolonged period of time. An exemplary adhesive patch composition can comprise a monolithic layer produced by mixing oxybutynin, pirenzepine, MT7, muscarinic receptor antagonist, and the like with a silicone-type adhesive or alternatively an acrylate-vinyl acetate adhesive in a solvent exemplified by methylene chloride, ethyl acetate, isopropyl myristate, and propylene glycol. The mixture would then be extruded onto a polyester-backing film to a uniform thickness of about 100 microns or greater with a precision wet-film applicator. The solvent is allowed to evaporate in a drying oven and the resulting "patch" is trimmed to the appropriate size.

The pharmaceutical for topical administration or alternatively for transdermal administration of an agent as described above (e.g., oxybutynin, pirenzepine, MT7, muscarinic receptor antagonist, and the like) may additionally incorporate a penetration enhancer and/or a thickening agent or gelling agent and/or an emollient and/or an antioxidant and/or an antimicrobial preservative and/or an emulsifying agent and/or a water miscible solvent and/or an alcohol and/or water.

According to one aspect, the pharmaceutical composition for topical administration or transdermal administration of an agent as described above (e.g., oxybutynin, pirenzepine, MT7, muscarinic receptor antagonist, and the like) may comprise one or more penetration enhancing agent or co-solvent for transdermal or topical delivery. A penetration enhancer is an excipient that aids in the diffusion of the active through the stratum corneum. Many penetration enhancers also function as co-solvents which are thought to increase the thermodynamic activity or solubility of the oxybutynin, pirenzepine, MT7, muscarinic receptor antagonist, and the like in the composition. Penetration enhancers are also known as accelerants, adjuvants or sorption promoters. A suitable penetration enhancer for use in the pharmaceutical compositions and methods described herein should: (i) be highly potent, with a specific mechanism of action; (ii) exhibit a rapid onset upon administration; (iii) have a predictable duration of action; (iv) have only non-permanent or reversible effects on the skin; (v) be chemically stable; (vi) have no or minimal pharmacological effects; (vii) be physically and chemically compatible with other composition components; (viii) be odorless; (ix) be colorless; (x) be hypoallergenic; (xi) be non-irritating; (xii) be non-phototoxic; (xiii) be non-comedogenic; (xiv) have a solubility parameter approximating that of the skin (10.5 cal/cm3); (xv) be readily available; (xvi) be inexpensive; and (xvii) be able to formulated in pharmaceutical compositions for topical or transdermal delivery of an active pharmaceutical agent.

Several classes of chemical compounds, with various mechanisms of action, can be used as penetration enhancers. Set forth below are non-limiting examples of penetration enhancing agents, many of which are also suitable co-solvents. Sulfoxides, such as dimethylsulfoxide and decylmethylsulfoxide can be used as penetration enhancing agents. Dimethylsulfoxide enhances penetration in part by increasing lipid fluidity and promoting drug partitioning. In contrast, decylmethylsulfoxide enhances penetration by reacting with proteins in the skin that change the conformation of the proteins, which results in the creation of aqueous channels.

Another class of penetration enhancers are alkanones, such as N-heptane, N-octane, N-nonane, N-decane, N-undecane, N-dodecane, N-tridecane, N-tetradecane and N-hexadecane. Alkanones are thought to enhance the penetration of an active agent by altering the stratum corneum. A further class of penetration enhancers are alkanol alcohols, such as ethanol, propanol, butanol, 2-butanol, pentanol, 2-pentanol, hexanol, octanol. nonanol, decanol and benzyl alcohol. Low molecular weight alkanol alcohols, i.e., those with 6 or less carbons, may enhance penetration in part by acting as solubilizing agents, while more hydrophobic alcohols may increase diffusion by extracting lipids from the stratum corneum. A further class of penetration enhancers are fatty alcohols, such as oleyl alcohol, caprylic alcohol, decyl alcohol, lauryl alcohol, 2-lauryl alcohol, myristyl alcohol, cetyl alcohol, stearyl alcohol, oleyl alcohol, linoleyl alcohol and linolenyl alcohol. Polyols, including propylene glycol, polyethylene glycol, ethylene glycol, diethylene glycol, triethylene glycol, dipropylene glycol, glycerol, propanediol, butanediol, pentanediol, hexanetriol, propylene glycol monolaurate and diethylene glycol monomethyl ether (transcutol), can also enhance penetration. Some polyols, such as propylene glycol, may function as a penetration enhancer by solvating alpha-kertin and occupying hydrogen bonding sites, thereby reducing the amount of active-tissue binding.

Another class of penetration enhancers are amides, including urea, dimethylacetamide, diethyltoluamide, dimethylformamide, dimethyloctamide, dimethyldecamide and biodegradable cyclic urea (e.g., 1-alkyl-4-imidazolin-2-one). Amides have various mechanisms of enhancing penetration. For example, some amides, such as urea increase the hydration of the stratum corneum, act as a keratolytic and create hydrophilic diffusion channels. In contrast, other amides, such as dimethylacetamide and dimethylformamide, increase the partition to keratin at low concentrations, while increasing lipid fluidity and disrupting lipid packaging at higher concentrations. Another class of penetration enhancing agents are pyrrolidone derivatives, such as 1-methyl-2-pyrrolidone, 2-pyrrolidone, 1-lauryl-2-pyrrolidone, 1-methyl-4-carboxy-2-pyrrolidone, 1-hexyl-4-carboxy-2-pyrrolidone, 1-lauryl-4-carboxy-2-pyrrolidone, 1-methyl-4-methoxycarbonyl-2-pyrrolidone, 1-hexyl-4-methoxycarbonyl-2-pyrrolidone, 1-lauryl-4-methoxycarbonyl-2-pyrrolidone, N-methyl-pyrrolidone, N-cyclohexylpyrrolidone, N-dimethylaminopropyl-pyrrolidone, N-cocoalkypyrrolidone and N-tallowalkypyrrolidone, as well as biodegradable pyrrolidone derivatives, including fatty acid esters of N-(2-hydroxyethyl)-2-pyrrolidone. In part, pyrrolidone derivatives enhance penetration through interactions with the keratin in the stratum corneum and lipids in the skin structure. An additional class of penetration enhancers are cyclic amides, including 1-dodecylazacycloheptane-2-one also known as AZONE® (AZONE is a registered trademark of Echo Therapuetics Inc., Philadelphia ,PA, USA), 1-geranylazacycloheptan-2-one, 1-farnesylazacycloheptan-2-one, 1-geranylgeranylazacycloheptan-2-one, 1-(3,7-dimethyloctyl)-azacycloheptan-2-one, 1-(3,7,1 1-trimefhyldodecyl)azacyclohaptan-2-one, 1-geranylazacyclohexane-2-one, 1-geranylazacyclopentan-2,5-dione and 1-famesylazacyclopentan-2-one. Cyclic amides, such as AZONE®, enhance the penetration of active agents in part by affecting the stratum corneum's lipid structure, increasing partitioning and increasing membrane fluidity.

Additional classes of penetration enhancers include diethanolamine, triethanolamine and hexamethylenlauramide and its derivatives.

Additional penetration enhancers include linear fatty acids, such as octanoic acid, linoleic acid, valeric acid, heptanoic acid, pelagonic acid, caproic acid, capric acid, lauric acid, myristric acid, stearic acid, oleic acid and caprylic acid. Linear fatty acids enhance penetration in part via selective perturbation of the intercellular lipid bilayers. In addition, some linear fatty acids, such as oleic acid, enhance penetration by decreasing the phase transition temperatures of the lipid, thereby increasing motional freedom or fluidity of the lipids. Branched fatty acids, including isovaleric acid, neopentanoic acid, neoheptanoic acid, nonanoic acid, trimethyl hexaonic acid, neodecanoic acid and isostearic acid, are a further class of penetration enhancers. An additional class of penetration enhancers are aliphatic fatty acid esters, such as ethyl oleate, isopropyl n-butyrate, isopropyl n-hexanoate, isopropyl n-decanoate, isopropyl myristate ("IPM"), isopropyl palmitate and octyldodecyl myristate. Aliphatic fatty acid esters enhance penetration by increasing diffusivity in the stratum corneum and/or the partition coefficient. In addition, certain aliphatic fatty acid esters, such as IPM, enhance penetration by directly acting on the stratum corneum and permeating into the liposome bilayers thereby increasing fluidity. Alkyl fatty acid esters, such as ethyl acetate, butyl acetate, methyl acetate, methyl valerate, methyl propionate, diethyl sebacate, ethyl oleate, butyl stearate and methyl laurate, can act as penetration enhancers. Alkyl fatty acid esters enhance penetration in part by increasing the lipid fluidity.

An additional class of penetration enhancers are anionic surfactants, including sodium laurate, sodium lauryl sulfate and sodium octyl sulfate. Anionic surfactants enhance penetration of active agents by altering the barrier function of the stratum corneum and allowing removal of water-soluble agents that normally act as plasticizers. A further class of penetration enhancers are cationic surfactants, such as cetyltrimethylammonium bromide, tetradecyltrimethylammonium, octyltrimethyl ammonium bromide, benzalkonium chloride, octadecyltrimethylammonium chloride, cetylpyridinium chloride, dodecyltrimethylammonium chloride and hexadecyltrimethylammonium chloride. Cationic surfactants enhance penetration by adsorbing at, and interacting with, interfaces of biological membranes, resulting in skin damage. A further class of penetration enhancers are zwitterionic surfactants, such as hexadecyl trimethyl ammoniopropane sulfonate, oleyl betaine, cocamidopropyl hydroxysultaine and cocamidopropyl betaine. Nonionic surfactants exemplified by Polyxamer 231, Polyxamer 182, Polyxamer 184, Polysorbate 20, Polysorbate 60, BRIJ® 30, BRIJ® 93, BRIJ® 96, BRIJ® 99 (BRIJ is a registered trademark of Brij Image & Information Inc., Greensboro, NC, USA), SPAN® 20, SPAN® 40, SPAN® 60, SPAN® 80, SPAN® 85 (SPAN is a registered trademark of Croda International PLC, East Yorkshire, UK), TWEEN® 20, TWEEN® 40, TWEEN® 60, TWEEN® 80 (TWEEN is a registered trademark of Uniqema Americas LLC, Wilmington, DE, USA), Myrj 45, MYRJ® 51, MYRJ® (MYRJ is a registered trademark of Uniqema Americas LLC, Wilmington, DE, USA), and MIGLYOL® 840 (MIGLYOL is a registered trademark of Cremer Oleo GMBH & Co., Hamburg, Fed. Rep. Germany), and the like. Nonionic surfactants enhance penetration in part by emulsifying the sebum and enhancing the thermodynamic activity or solubility of the active.

Another class of penetration enhancer increase the thermodynamic activity or solubility of the active, which include, but are not limited to, n-octanol, sodium oleate, D-limonene, monoolein, cineol, oleyl oleate, and isopropryl myristate.

Other penetration enhancers are bile salts, such as sodium cholate, sodium salts of taurocholic acid, glycolic acids and desoxycholic acids. Lecithin also has been found to have penetration enhancing characteristics. An additional class of penetration enhancers are terpenes, which include hydrocarbons, such as d-limonene, alpha-pinene and beta-carene; alcohols, such as, alpha-terpineol, terpinen-4-ol and carvol; ketones, such ascarvone, pulegone, piperitone and menthone; oxides, such as cyclohexene oxide, limonene oxide, alpha-pinene oxide, cyclopentene oxide and 1,8-cineole; and oils such as ylang ylang, anise, chenopodium and eucalyptus. Terpenes enhance penetration in part by disrupting the intercellular lipid bilayer to increase diffusivity of the active and opening polar pathways within and across the stratum corneum. Organic acids, such as salicylic acid and salicylates (including their methyl, ethyl and propyl glycol derivates), citric acid and succinic acid, are penetration enhancers. Another class of penetration enhancers are cyclodextrins, including 2-hydroxypropyl-beta-cyclodextrin and 2,6-dimethyl-beta-cyclodextrin. Cyclodextrins enhance the permeation of active agents by forming inclusion complexes with lipophilic actives and increasing their solubility in aqueous solutions.

The penetration enhancing agent(s) and/or co-solvent(s) is/are present in the pharmaceutical composition for topical administration or transdermal administration of an agent as described above (e.g., oxybutynin, pirenzepine, MT7, muscarinic receptor antagonist, and the like) in an amount sufficient to provide the desired level of drug transport through the stratum corneum and epidermis or to increase the thermodynamic activity or solubility of the oxybutynin, pirenzepine, MT7, muscarinic receptor antagonist, and the like. The one or more pharmaceutically acceptable penetration enhancer and/or co-solvent may be present in a total amount by weight of about 0.1%, about 0.2%, about 0.3%, about 0.4%, about 0.5%, about 0.6%, about 0.7%, about 0.8%, about 0.9%, about 1.0%, about 1.1%, about 1.2%, about 1.3%, about 1.4%, about 1..5%, about 1..6%, about 1.7%, about 1.8%, about 1.9%, about 2.0%, about 2.1%, about 2..2%, about 2..3%, about 2.4%, about 2.5%, about 2.6%, about 2.7%, about 2.8%, about 2, .9%, about 3..0%, about 3.1%, about 3.2%, about 3.3%, about 3.4%, about 3.5%, about 3 .6%, about 3 .7%, about 3.8%, about 3.9%, about 4.0%, about 4.1%, about 4.2%, about 4..3%, about 4 .4%, about 4.5%, about 4.6%, about 4.7%, about 4.8%, about 4.9%, about 5..0%, about 5 .1 %, about 5.2%, about 5.3%, about 5.4%, about 5.5%, about 5.6%, about 5..7%, about 5, .8%, about 5.9%, about 6.0%, about 6.1%, about 6.2%, about 6.3%, about 6..4%, about 6, .5%, about 6.6%, about 6.7%, about 6.8%, about 6.9%, about 7.0%, about 7..1%, about 7 .2%, about 7.3%, about 7.4%, about 7.5%, about 7.6%, about 7.7%, about 7..8%, about 7..9%, about 8.0%, about 8.1%, about 8.2%, about 8.3%, about 8.4%, about 8..5%, about 8..6%, about 8.7%, about 8.8%, about 8.9%, about 9.0%, about 9.1%, about 9..2%, about 9..3%, about 9.4%, about 9.5%, about 9.6%, about 9.7%, about 9.8%, about 9.9% or about 10%, about 11%, about 12%, about 13%, about 14%, about 15%, about 16%, about 17%, about 18%, about 1%, about 20%, about 21%, about 22%, about 23%, about 24%, about 25%, about 26%, about 27%, about 28%, about 29%, about 30%, about 31%, about 32%, about 33%, about 34%, about 35%, about 36%, about 37%, about 38%, about 39%, about 40%, about 41%, about 42%, about 43%, about 44%., about 45%, about 46%, about 47%, about 48%, about 49%, about 50%, about 51%, about 52%, about 53%, about 54%, about 55%, about 56%, about 57%, about 58%, about 59%, about 60%, about 61 %, about 62%, about 63%, about 64%, about 65%, about 66%, about 67%, about 68%, about 69%, about 70%, about 71%, about 72%, about 73%, about 74%, about 75%, about 76%, about 77%, about 78%, about 79%, about 80%, about 81%, about 82%, about 83%, about 84%, about 85%, about 86%, about 87%, about 88%, about 89%, about 90%, about 91 %, about 92%, about 93%, about 94%, or about 95%.

The selected penetration enhancer should be pharmacologically inert, non-toxic, and non-allergenic, have rapid and reversible onset of action, and be compatible with the compositions of the invention. Examples of penetration enhancers exemplified by transcutol P, ethyl alcohol, isopropyl alcohol, lauryl alcohol, salicylic acid, octolyphenylpolyethylene glycol, polyethylene glycol 400, propylene glycol, N-decylmethylsulfoxide, DMSO and azacyclo compounds.

In one exemplary embodiment, the present disclosure pertains to compositions for local administration of a muscarinic acetylcholine receptor antagonist(s) in a pharmaceutically sufficient amount to treat peripheral neuropathy. As used herein, the term "local" refers to the limited area near the site of administration, generally the nerves at or near skin including the epidermis, the dermis, the dermatomes and the like, with no or limited systemic penetration beyond the skin.

Preferably, the topical delivery is designed to maximize drug delivery through the stratum corneum and into the epidermis or dermis or dermatome, and to minimize absorption into the circulatory system. More preferable are agents that may be used in topical formulations to prevent the passage of active ingredients or excipients into the lower skin layers. These so-called skin retardants have been readily developed for many over-the-counter (OTC) skin formulations, such as sunscreens and pesticides, where the site of action is restricted to the skin surface or upper skin layers. Research in the area of permeation enhancement or retardation is yielding valuable insights into the structure-activity relationships of enhancers as well as retardants (Asbill et al., 2000, Percutaneous penetration enhancers: local versus transdermal activity. Pharm. Sci. Tech. Today, 3(1):36-41; Kaushik, et al., 2008, Percutaneous permeation modifiers: enhancement versus retardation. Exp. Opin. Drug Del. 5(5):517-529; Trommer et al., 2006, Overcoming the Stratum Corneum: The Modulation of Skin Penetration. Skin Pharmacol. Physiol. 19:106-121) including such compounds as ketorolac stearate, Aminocaprolactam Analogues, Dicarboxylic acid ester, sodium citrate, and the like.

The compositions described herein can further comprise components usually admixed in such preparations. For example, the compositions may also include additional ingredients such as other carriers, moisturizers, oils, fats, waxes, surfactants, thickening agents, antioxidants, viscosity stabilizers, chelating agents, buffers, preservatives, perfumes, dyestuffs, lower alkanols, humectants, emollients, dispersants, sunscreens such as radiation blocking compounds or particularly UV-blockers, antibacterials, antifungals, disinfectants, vitamins, antibiotics, or other anti-acne agents, as well as other suitable materials that do not have a significant adverse effect on the activity of the topical composition. Additional ingredients for inclusion in the carrier are sodium acid phosphate moisturizer, witch hazel extract carrier, glycerin humectant, apricot kernel oil emollient, corn oil dispersant, and the like which are further detailed below. Those of skill in the art will readily recognize additional ingredients, which can be admixed in the compositions described herein.

According to another aspect, the pharmaceutical composition for topical administration or for transdermal application of oxybutynin, pirenzepine, MT7, muscarinic receptor antagonist, and the like may comprise a thickening or gelling agent suitable for use in the compositions and methods described herein to increase the viscosity of the composition. Suitable agents (also known as gelling agents) are exemplified neutralized anionic polymers or neutralized carbomers, such as polyacrylic acid, carboxypolymethylene, carboxymethyl cellulose and the like, including derivatives of Ultrez 10, CARBOPOL® polymers, such as CARBOPOL® 940, CARBOPOL® 941 , CARBOPOL® 954, CARBOPOL® 980, CARBOPOL® 981, CARBOPOL® ETD 2001 , CARBOPOL® EZ-2 and CARBOPOL® EZ-3. (CARBOPOL is a registered trademark of Lubrizol Advanced Materials Inc., Cleveland, OH, USA). As used herein, a "neutralized carbomer" is a synthetic, high molecular weight polymer, composed primarily of a neutralized polyacrylic acid. Further, when a base is added to neutralize a carbomer solution, the viscosity of the solution increases. Also suitable are other known polymeric thickening agents, such as PEMULEN® polymeric emulsifiers, NOVEON® polycarbophils (PEMULEN and NOVEON are registered trademarks of Lubrizol Advanced Materials Inc.), and KLUCEL® (KLUCEL is a registered trademark of Hercules Inc., Wilmington, DE, USA). Additional thickening agents, enhancers and adjuvants may generally be found in Remington's The Science and Practice of Pharmacy as well as in the Handbook of Pharmaceutical Excipients (Arthur H. Kibbe ed. 2000). Alternatively, the pharmaceutical composition for topical administration or for transdermal application of oxybutynin, pirenzepine, MT7, muscarinic receptor antagonist, and the like may comprise an anionic polymer thickening agent precursor, such as a carbomer, which has been combined with a neutralizer in an amount sufficient to form a gel or gel-like composition with a viscosity greater than 1000 cps as measured by a Brookfield RV DVII+ Viscometer with spindle CPE-52, torque greater than 10% and the temperature maintained at 25° C. Alternatively, the anionic polymer thickening agent precursor may be combined with a neutralizer selected from the group consisting of: sodium hydroxide, ammonium hydroxide, potassium hydroxide, arginine, aminomethy] propanol, tetrahydroxypropyl ethylenediamine, triethanolamine ("TEA"), tromethamine, PEG-15 cocamine, diisopropanolamine, and triisopropanolamine, or combinations thereof in an amount sufficient to neutralize the anionic polymer thickening agent precursor to form a gel or gel-like composition in the course of forming the composition. The thickening agents or gelling agents are present in an amount sufficient to provide the desired rheological properties of the composition, which include having a sufficient viscosity for forming a gel or gel-like composition that can be applied to the skin of a mammal. The thickening agent or gelling agent is present in a total amount by weight of about 0.1%, about 0.25%, about 0.5%, about 0.75%, about 1%, about 1.25%, about 1.5%, about 1.75%, about 2.0%, about 2.25%, about 2.5%, about 2.75%, about 3.0%, about 3.25%, about 3.5%, about 3.75%, about 4.0%, about 4.25%, about 4.5%, about 4.75%, about 5.0%, about 5.25%, about 5.5%, about 5.75%, about 6.0%, about 6.25%, about 6.5%, about 6.75%, about 7.0%, about 7.25%, about 7.5%, about 7.75%, about 8.0%, about 8.25%, about 8.5%o, about 8.75%), about 9.0%, about 9.25%, about 9.5%, about 9.75%, about 10%, about 1 1 %, about 11.5%, about 12%, about 12.5%, about 13%, about 13.5%, about 14%, about 14.5% or about 15%, and therebetween.

According to another aspect, the pharmaceutical composition for topical administration or for transdermal application of oxybutynin, pirenzepine, MT7, muscarinic receptor antagonist, and the like may comprise an emollient. Suitable emollients are exemplified by mineral oil, mixtures of mineral oil and lanolin alcohols, cetyl alcohol, cetostearyl alcohol, petrolatum, petrolatum and lanolin alcohols, cetyl esters wax, cholesterol, glycerin, glyceryl monostearate, isopropyl myristate, isopropyl palmitate, lecithin, allyl caproate, althea officinalis extract, arachidyl alcohol, argobase EUC, butylene glycol, dicaprylate/dicaprate, acacia, allantoin, carrageenan, cetyl dimethicone, cyclome hicone, diethyl succinate, dihydroabietyl behenate, dioctyl adipate, ethyl laurate, ethyl palmitate, ethyl stearate, isoamyl laurate, octanoate, PEG-75, lanolin, sorbitan laurate, walnut oil, wheat germ oil, super refined almond, super refined sesame, super refined soyabean, octyl palmitate, caprylic/capric triglyceride and glyceryl cocoate. An emollient, if present, is present in the compositions described herein in an amount by weight of the composition of about 1% to about 30%, about 3% to about 25%, or about 5% to about 15%. Illustratively, one or more emollients are present in a total amount of about 1% by weight, about 2%, about 3%, about 4%, about 5%, about 6%, about 7%., about 8%, about 9%, about 10%, about 1 1%, about 12%, about 13%, about 14%, about 1 %, about 16%, about 17%, about 18%, about 19%, about 20%, about 21%, about 22%, about 23%, about 24%, about 25%, about 26%, about 27%, about 28%, about 29%, or about 30%, and therebetween.

According to another aspect, the pharmaceutical composition for topical administration or for transdermal application of oxybutynin, pirenzepine, MT7, muscarinic receptor antagonist, and the like may comprise an antioxidant. Suitable antioxidants are exemplified by citric acid, butylated hydroxytoluene (BHT), ascorbic acid, glutathione, retinol, a-tocopherol, β-carotene, a-carotene, ubiquinone, butylated hydroxyanisole, ethyl enediaminetetraacetic acid, selenium, zinc, lignan, uric acid, lipoic acid, and N-acetylcysteine. An antioxidant, if present, is present in the compositions described herein in a total amount selected from the range of about 0.025% to about 1.0% by weight.

According to another aspect, the pharmaceutical composition for topical administration or for transdermal application of oxybutynin, pirenzepine, MT7, muscarinic receptor antagonist, and the like may comprise an antimicrobial preservative. Illustrative anti-microbial preservatives include acids, including but not limited to, benzoic acid, phenolic acid, sorbic acids, alcohols, benzethonium chloride, bronopol, butylparaben, cetrimide, chlorhexidine, chlorobutanol, chlorocresol, cresol, ethylparaben, imidurea, methylparaben, phenol, phenoxyethanol, phenylethyl alcohol, phenylmercuric acetate, phenylmercuric borate, phenylmercuric nitrate, potassium sorbate, propylparaben, sodium propionate or thimerosal. The anti-microbial preservative, if present, is present in an amount by weight of the composition of about 0.1 % to about 5%, about 0.2% to about 3%, or about 0.3% to about 2%, for example about 0.2%, about 0.4%, about 0.6%, about 0.8%, about 1%, about 1.2%, about 1.4%, about 1.6%, about 1.8%, about 2%, about 2.2%, about 2.4%, about 2.6%, about 2.8%, about 3.0%, about 3.2%, about 3.4%, about 3.6%, about 3.8%, about 4%, about 4.2%, about 4.4%, about 4.6%, about 4.8%, or about 5%.

According to another aspect, the pharmaceutical composition for topical administration or for transdermal application of oxybutynin, pirenzepine, MT7, muscarinic receptor antagonist, and the like may comprise one or more emulsifying agents. The term "emulsifying agent" refers to an agent capable of lowering surface tension between a nonpolar and polar phase and includes self emulsifying agents. Suitable emulsifying agents can come from any class of pharmaceutically acceptable emulsifying agents exemplified by carbohydrates, proteins, high molecular weight alcohols, wetting agents, waxes and finely divided solids. The optional emulsifying agent, if present, is present in a composition in a total amount of about 1% to about 25%, about 1% to about 20%, or about 1% to about 15% by weight of the composition. Illustratively, one or more emulsifying agents are present in a total amount by weight of about 1%, about 2%, about 3%, about 4%, about 5%, about 6%, about 7%, about 8%, about 9%, about 10%, about 1 1%, about 12%, about 13%, about 14%, about 15%, about 16%, about 17%, about 18%, about 19%, about 20%, about 21%, about 22%, about 23%, about 24%, or about 25%.

According to another aspect, the pharmaceutical composition for topical administration or for transdermal application of oxybutynin, pirenzepine, MT7, muscarinic receptor antagonist, and the like may comprise a water miscible solvent exemplified by propylene glycol. A suitable water miscible solvent refers to any solvent that is acceptable for use in a pharmaceutical composition and is miscible with water. If present, the water miscible solvent is present in a composition in a total amount of about 1 % to about 95%, about 2% to about 75%, about 3% to about 50%, about 4% to about 40%, or about 5% to about 25% by weight of the composition.

According to another aspect, the pharmaceutical composition for topical administration or for transdermal application of oxybutynin, pirenzepine, MT7, muscarinic receptor antagonist, and the like may comprise one or more alcohols. In a further embodiment, the alcohol is a lower alcohol. As used herein, the term "lower alcohol," alone or in combination, means a straight-chain or branched-chain alcohol moiety containing one to about six carbon atoms. In one embodiment, the lower alcohol contains one to about four carbon atoms, and in another embodiment the lower alcohol contains two or three carbon atoms. Examples of such alcohol moieties include methanol, ethanol, ethanol USP (i.e., 95% v/v), n-propanol, isopropanol, n-butanol, isobutanol, sec-butanol, and tert-butanol. As used herein, the term "ethanol" refers to C2H5OH. It may be used as dehydrated alcohol USP, alcohol USP or in any common form including in combination with various amounts of water. If present, the alcohol is present in an amount sufficient to form a composition which is suitable for contact with a mammal. For example, in a total amount by weight of about 1 %, about 2%, about 3%, about 4%, about 5%, about 6%, about 7%, about 8%, about 9%, about 10%, about 1 1%, about 12%, about 13%, about 14%, about 15%, about 16%, about 17%, about 18%, about 19%, about 20%, about 21%, about 22%, about 23%, about 24%, about 25%.

According to another aspect, the pharmaceutical composition for topical administration or for transdermal application of oxybutynin, pirenzepine, MT7, muscarinic receptor antagonist, and the like may comprise water separately in a quantity or amount sufficient to achieve the desired weight of the pharmaceutical composition.

Oxybutynin, pirenzepine, MT7, muscarinic receptor antagonist, and the like comprises about 0.1%, about 0.2%, about 0.3%, about 0.4%, about 0.5%, about 0.6%, about 0.7%, about 0.8%, about 0.9%, about 1%, about 1.1%, about 1.2%, about 1.3%, about 1.4%, about 1.5%, about 1.6%, about 1.7%, about 1.8%, about 1.9%, about 2%, about 2.1%, about 2.2%, about 2.3%, about 2.4%, about 2.5%, about 2.6%, about 2.7%, about 2.8%, about 2.9%, about 3%, about 3.1 %, about 3.2%, about 3.3%, about 3.4%, about 3.5%, about 3.6%, about 3.7%, about 3.8%, about 3.9%, about 4%, about 4.1%, about 4.2%, about 4.3%, about 4.4%, about 4.5%, about 4.6%, about 4.7%, about 4.8%, about 4.9%, about 5%, about 5.1%, about 5.2%, about 5.3%, about 5.4%, about 5.5%, about 5.6%, about 5.7%, about 5.8%, about 5.9%, about 6%, about 6.1%, about 6.2%, about 6.3%, about 6.4%, about 6.5%, about 6.6%, about 6.7%, about 6.8%, about 6.9%, about 7%, about 7.1%, about 7.2%, about 7.3%, about 7.4%, about 7.5%, about 7.6%, about 7.7%, about 7.8%, about 7.9%, about 8%, about 8.1%, about 8.2%, about 8.3%, about 8.4%, about 8.5%, about 8.6%, about 8.7%, about 8.8%, about 8.9%, about 9%, about 9.1%, about 9.2%, about 9.3%, about 9.4%, about 9.5%, about 9.6%, about 9.7%, about 9.8%, about 9.9%, about 10%, about 11 %, about 12%, about 13%, about 14%, about 15%, about 16%, about 17%, about 18%, about 19%, about 20%, about 21%, about 22%, about 23%, about 24%o, about 25%o, about 26%, about 27%, about 28%, about 29%, about 30%, about 31%, about 32%, about 33%, about 34%, about 35%, about 36%, about 37%, about 38%, about 39%, about 40%, about 41%, about 42%, about 43%, about 44%, about 45%, about 46%, about 47%, about 48%, about 49%, about 50%, about 55%, about 60%, about 65%, about 70%, about 75%, about 80%, about 85%, about 90% or about 95% by weight of the pharmaceutical composition for topical application or for transdermal application.

Another embodiment pertains to pharmaceutical compositions comprising oxybutynin, pirenzepine, MT7, muscarinic receptor antagonist, and the like formulated for parenteral administration by injection. The injectable pharmaceutical compositions of the present disclosure comprise a suitable carrier solution exemplified by sterile water, saline, and buffered solutions at physiological pH. Suitable buffered solutions are exemplified by Ringer's dextrose solution and Ringer's lactated solutions. The carrier solution may comprise in a total amount by weight of about 0.1%, about 0.2%, about 0.3%, about 0.4%, about 0.5%, about 0.6%, about 0.7%, about 0.8%, about 0.9%, about 1.0%, about 1.1 %, about 1.2%, about 1.3%, about 1.4%, about 1.5%, about 1.6%, about 1.7%, about 1.8%, about 1.9%, about 2.0%>, about 2.1%, about 2.2%, about 2.3%, about 2.4%, about 2.5%, about 2.6%, about 2.7%, about 2.8%, about 2.9%, about 3.0%, about 3.1%, about 3.2%, about 3.3%, about 3.4%, about 3.5%, about 3.6%, about 3.7%, about 3.8%, about 3.9%, about 4.0%, about 4.1%, about 4.2%, about 4.3%, about 4.4%, about 4.5%, about 4.6%, about 4.7%, about 4.8%, about 4.9%, about 5.0%, about 5.1%, about 5.2%, about 5.3%, about 5.4%, about 5.5%, about 5.6%, about 5.7%, about 5.8%, about 5.9%, about 6.0%, about 6.1%, about 6.2%, about 6.3%>, about 6.4%, about 6.5%, about 6.6%, about 6.7%, about 6.8%, about 6.9%, about 7.0%, about 7.1%, about 7.2%, about 7.3%, about 7.4%, about 7.5%, about 7.6%, about 7.7%, about 7.8%, about 7.9%, about 8.0%, about 8.1%, about 8.2%, about 8.3%, about 8.4%, about 8.5%, about 8.6%, about 8.7%, about 8.8%, about 8.9%, about 9.0%, about 9.1%, about 9.2%), about 9.3%, about 9.4%, about 9.5%, about 9.6%, about 9.7%, about 9.8%, about 9.9% or about 10%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, about 30%, about 31%o, about 32%, about 33%, about 34%, about 35%, about 36%, about 37%, about 38%, about 39%, about 40%, about 41%, about 42%, about 43%, about 44%, about 45%, about 46%, about 47%, about 48%, about 49%, about 50%, about 51 %, about 52%, about 53%, about 54%, about 55%, about 56%, about 57%, about 58%, about 59%, about 60%, about 61%, about 62%, about 63%», about 64%, about 65%, about 66%, about 67%, about 68%, about 69%, about 70%, about 71%, about 72%, about 73%, about 74%, about 75%, about 76%, about 77%, about 78%, about 79%, about 80%, about 81%, about 82%, about 83%, about 84%, about 85%, about 86%, about 87%, about 88%, about 89%, about 90%, about 91%, about 92%, about 93%, about 94%, or about 95%.

According to one aspect, the injectable pharmaceutical compositions may additionally incorporate one or more non-aqueous solvents exemplified by propylene glycol, polyethylene glycol, vegetable oils such as olive oil, and injectable organic esters exemplified by ethyl oleate.

According to another aspect, the injectable pharmaceutical compositions may additionally incorporate one or more of antimicrobials, anti-oxidants, chelating agents and the like.

The injectable pharmaceutical compositions may be presented in unit-dose or multidose containers exemplified by sealed ampules and vials. The injectable pharmaceutical compositions may be stored in a freeze-dried (lyophilized) condition requiring the addition of a sterile liquid carrier, e.g., sterile saline solution for injections, immediately prior to use.

Another embodiment pertains to pharmaceutical compositions comprising oxybutynin, pirenzepine, MT7, muscarinic receptor antagonist, and the like formulated for oral administration. The oral pharmaceutical compositions may be provided as capsules or tablets; as powders or granules; as solutions, syrups or suspensions (in aqueous or non-aqueous liquids). Tablets or hard gelatine capsules may comprise, for example, lactose, starch or derivatives thereof, magnesium stearate, sodium saccharine, cellulose, magnesium carbonate, stearic acid or salts thereof. Soft gelatine capsules may comprise, for example, vegetable oils, waxes, fats, semisolid, or liquid polyols, etc. Solutions and syrups may comprise, for example, water, polyols and sugars. The oxybutynin, pirenzepine, MT7, muscarinic receptor antagonist, and the like may be coated with or admixed with a material (e.g., glyceryl monostearate or glyceryl distearate) that delays disintegration or affects absorption of the active agent in the gastrointestinal tract. Thus, for example, the sustained release of an active agent may be achieved over many hours and, if necessary, the active agent can be protected from being degraded within the gastrointestinal tract. Taking advantage of the various pH and enzymatic conditions along the gastrointestinal tract, pharmaceutical compositions for oral administration may be formulated to facilitate release of an active agent at a particular gastrointestinal location.

The pharmaceutical compositions described herein are used in a "pharmacologically effective amount." A "pharmacologically effective amount" is the amount of oxybutynin, pirenzepine, MT7, muscarinic receptor antagonist, and the like in the composition which is sufficient to deliver a therapeutic amount of the active agent during the dosing interval in which the pharmaceutical composition is administered. Accordingly, the amount of the pharmaceutical composition administered to deliver a therapeutically effective amount of oxybutynin, pirenzepine, MT7, muscarinic receptor antagonist, and the like is about 0.01 g, about 0.05 g, about 0.1 g, about 0.2 g, about 0.3 g, about 0.4 g, about 0.5 g, about 0.6 g, about 0.7 g, about 0.8 g, about 0.9 g, about 1 g, about 1.1 g, about 1.2 g, about 1.3 g, about 1.4 g, about 1.5 g, about 1.6 g, about 1.7 g, about 1.8 g, about 1.9 g, about 2 g, about 2.1 g, about 2.2 g, about 2.3 g, about 2.4 g, about 2.5 g, about 2.6 g, about 2.7 g, about 2.8 g, about 2.9 g, about 3 g, about 3.1 g, about 3.2 g, about 3.3 g, about 3.4 g, about 3.5 g, about 3.6 g, about 3.7 g, about 3.8 g, about 3.9 g, about 4 g, about 4.1 g, about 4.2 g, about 4.3 g, about 4.4 g, about 4.5 g, about 4.6 g, about 4.7 g, about 4.8 g, about 4.9 g, about 5 g, about 5.1 g, about 5.2 g, about 5.3 g, about 5.4 g, about 5.5 g, about 5.6 g, about 5.7 g, about 5.8 g, about 5.9 g, about 6 g, about 6.1 g, about 6.2 g, about 6.3 g, about 6.4 g, about 6.5 g, about 6.6 g, about 6.7 g, about 6.8 g, about 6.9 g, about 7 g, about 7.1 g, about 7.2 g, about 7.3 g, about 7.4 g, about 7.5 g, about 7.6 g, about 7.7 g, about 7.8 g, about 7.9 g, about 8 g, about 8.1 g, about 8.2 g, about 8.3 g, about 8.4 g, about 8.5 g, about 8.6 g, about 8.7 g, about 8.8 g, about 8.9 g, about 9 g, about 9.1 g, about 9.2 g, about 9.3 g, about 9.4 g, about 9.5 g, about 9.6 g, about 9.7 g, about 9.8 g, about 9.9 g or about 10 g.

The following examples are provided to more fully describe the disclosure and are presented for non-limiting illustrative purposes.

### EXAMPLES

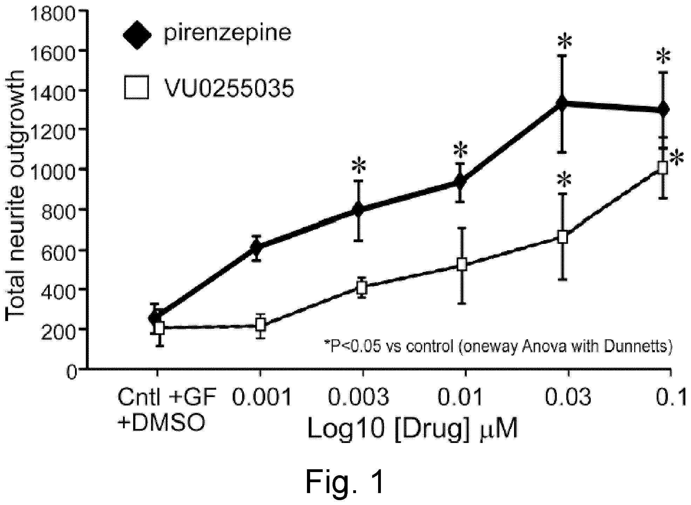

The fundamental hypothesis is that blocking endogenous cholinergic constraint using a muscarinic receptor antagonist will promote re-growth of neurons in patients with established peripheral neuropathy. This occurs via activation of AMPK and PGC-1α and the subsequent augmentation of mitochondrial function. This pathway comprises a proximal step involving receptor antagonist-dependent activation of CaMKKβ (or CaMKK2). This hypothesis is supported by extensive preclinical data using *in vitro* and *in vivo* model systems. Experiments show that muscarinic receptor antagonists dose-dependently promote neurite outgrowth from sensory neurons derived from adult rodents (Fig. 1). A specific requirement for M1 receptor antagonism is confirmed by the increased neurite outgrowth from sensory neurons derived from M1R KO mice.

### Example 1:

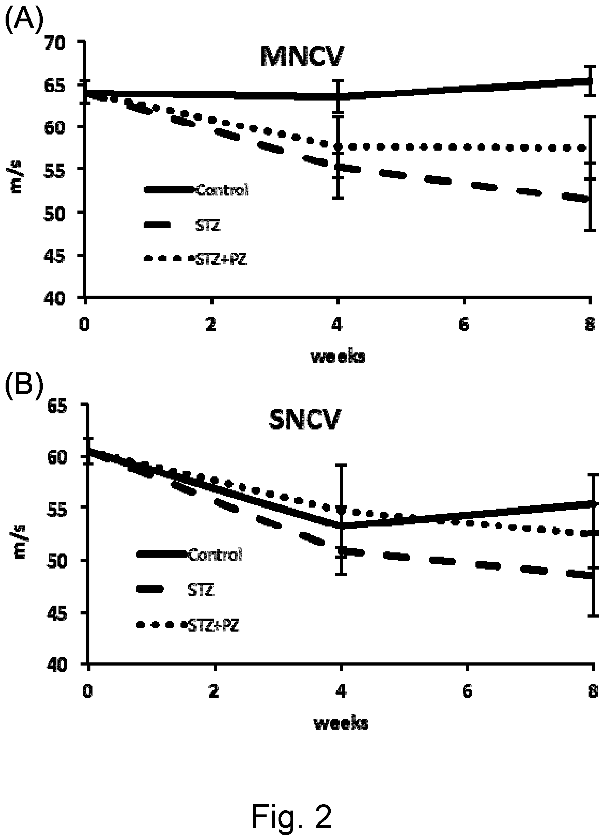

In previous work, we showed that pirenzepine can prevent and reverse small fiber neuropathy in diabetes. Now we demonstrate that pirenzepine can also prevent large fiber motor nerve conduction velocity (MNVC) slowing (Fig. 2(A)) and sensory nerve conduction velocity (SNCV) slowing (Fig. 2(B)) in streptozotocin-induced (STZ-induced) diabetic rats. The STZ rat is a model of type 1 diabetes. Rats were treated with 5 mg/kg (s.c.) pirenzepine for 8 weeks following the method taught by Calcutt et al. (2003, Therapeutic efficacy of sonic hedgehog protein in experimental diabetic neuropathy. J. Clin. Invest. 111:507-514). Nerve conduction velocities (NCV) were determined following the methods taught by Mizisin et al. (2004, Ciliary Neurotrophic Factor Improves Nerve Conduction and Ameliorates Regeneration Deficits in Diabetic Rats. Diabetes 53:1807-12). The data show that diabetes caused time-dependent slowing of large fiber motor and sensory NCV (both p<0.01 vs control at 8 weeks of diabetes) and these disorders were attenuated by pirenzepine treatment (both p<0.05 vs vehicle-treated diabetic rats). All data are group mean±SEM (N=5-6 group). Statistical analysis by one-way ANOVA with Dunnett's post-hoc test. These date demonstrate that pirenzepine is able to protect function of large-diameter myelinated fibers of both motor and sensory nerves.

### Example 2:

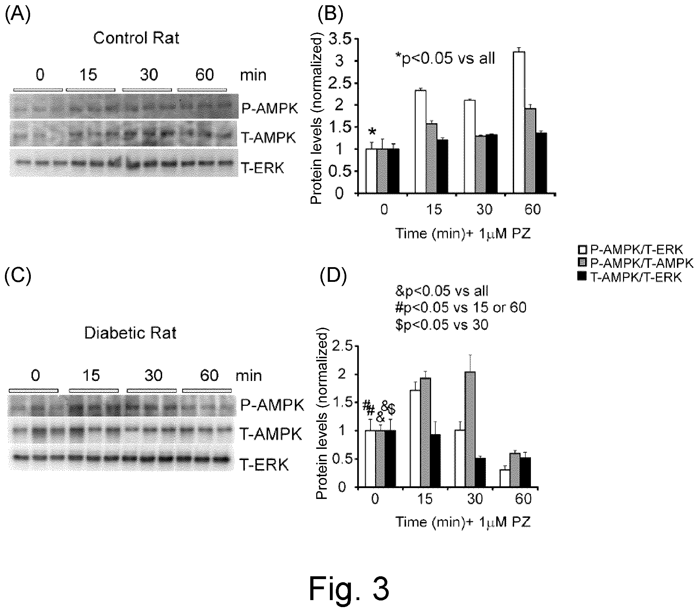

Subsets of cultured adult sensory neurons derived from age-matched control rats or 3-5 month STZ-induced diabetic rats were treated with a 1.0 µM pirenzepine-HCl dosage. The first subset was processed immediately while the second, third and fourth subsets of the neurons from the control rats and the diabetic rats were processed after 15 min, after 30 min, and after 60 min respectively following treatment with pirenzepine. The cell cultures were lysed and then used to prepare Western blots which were then probed with antibodies against phosphorylated AMPK (P-AMPK), total AMPK (T-AMPK) and total ERK (T-ERK). Fig. 3(A) shows the gels produced from the cultures from age-matched control rats while Fig. 3(C) shows the gels produced from the cultures from diabetic rats. The data shown in in Figs. 3(B) and 3(D), where the Y-axis represents levels of protein quantified from the blots in (A) and (C) and normalized to control, show that pirenzepine significantly activated P-AMPK in cultures from normal rats (Fig. 3(B)) and in type 1 diabetic rats (Fig. 3(D)). Values are means ± SEM, N=3/group. Statistical significance was analyzed using one-way ANOVA with Tukey's post-hoc test.

### Example 3:

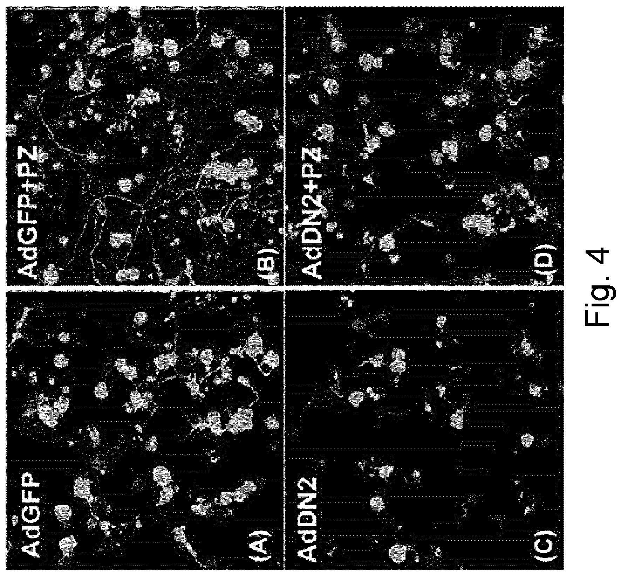

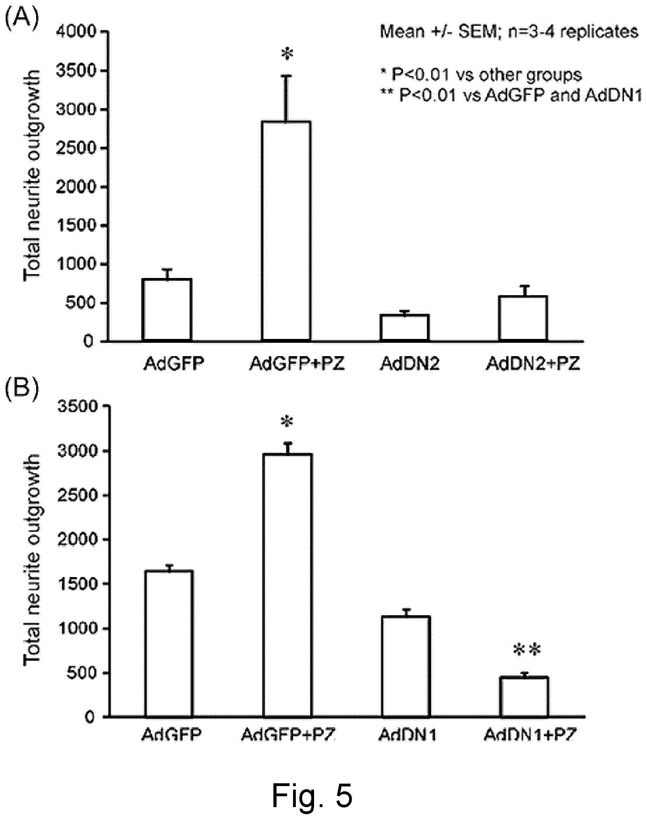

Adult sensory neuron cultures derived from STZ-induced diabetic rats were transduced for two days after treatment with 1.0 µM pirenzepine, with two adenovirus over-expressing dominant negative mutants of AMPK, i.e., AMPK subunit α1 and α2 mutants named "AdDN1" and "AdDN2" (the adenovirus over-expressing dominant negative mutants were gifts from Dr. Jason Dyck, University of Alberta) following the procedure taught by Roy Chowdhury et al., (2012). Figs. 4(A) and 4(B) are micrographs of GFP fluorescence from adult rat sensory neuron cultures transduced with the control adenoviral vector expressing only GFP wherein Fig. 4(A) did not receive a pirenzepine treatment whereas Fig. 4(B) did. Figs. 4(C) and 4(D) are micrographs of GFP fluorescence from adult rat sensory neuron cultures transduced with the "AdDN" dominant negative AMPK mutant wherein Fig. 4(C) did not receive a pirenzepine treatment whereas Fig. 4(D) did. The data in Figs. 5(A) and 5(B) show that blockage of AMPK signaling using adenovirus-delivered dominant negative mutants completely inhibited pirenzepine-induced neurite outgrowth.

### Example 4:

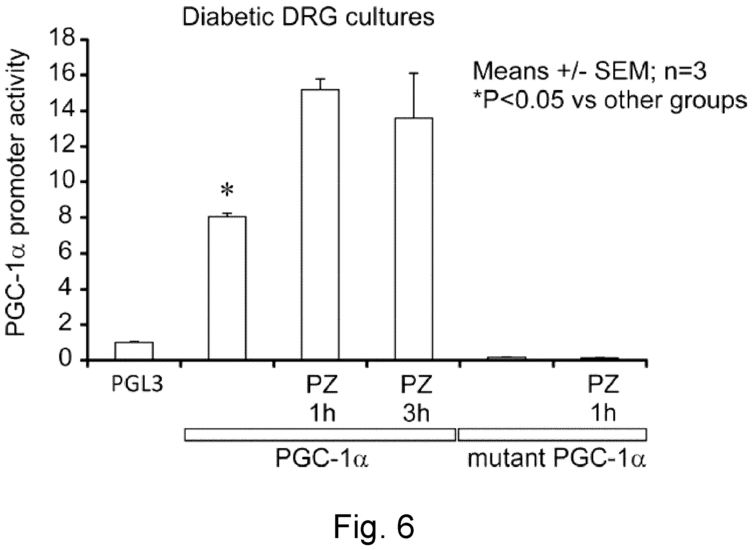

PGC-la transcriptional activity was determined in dissociated adult sensory neurons derived from STZ-induced diabetic rats by transfection for two days with luciferase-based reporter plasmids by following the methods taught in the AMAXA® NUCLEOFECTOR® II Manual (www. amaxa. com; AMAX and NUCLEOFECTOR are registered trademarks of Amaxa GmBH Corp., Koln, Fed Rep. Germany) and by Saleh et al. (2013, Ciliary neurotrophic factor activates NF-κB to enhance mitochondrial bioenergetics and prevent neuropathy in sensory neurons of streptozotocin-induced diabetic rodents. Neuropharmacology 65: 65-75) (the luciferase-based reporter plasmids were a gift from Dr. Michael Czubryt, University of Manitoba). Fig. 6 shows that 1µM pirenzepine enhanced transcriptional activity of PGC-la in cultured adult sensory neurons derived from STZ-induced diabetic rats (means ± SEM of N=3 replicate cultures. Oneway ANOVA with Tukey's postdoc test. Values normalized to control reporter (PGL3) expression). However, mutant PGC-la plasmids exhibited zero activity in this assay. Accordingly, these data demonstrate that pirenzepine signals via activation of the AMPK/PGC-1α axis to drive neurite outgrowth.

### Example 5:

Mitochondrial respiration in adult mouse sensory neuron cultures from wild type control mice, M1 receptor knockout mice (M1R KO), and from diabetic mice that received dosing with 1 µM of VU0255035 for 3 h, were assessed following the methods taught by Roy Chowdhury et al. (2012). The M1R knockout mice were a gift from Dr. Jurgen Wess (Molecular Signaling Section, Laboratory of Bioorganic Chemistry, National Institute of Diabetes and Digestive and Kidney Diseases, National Institutes of Health).

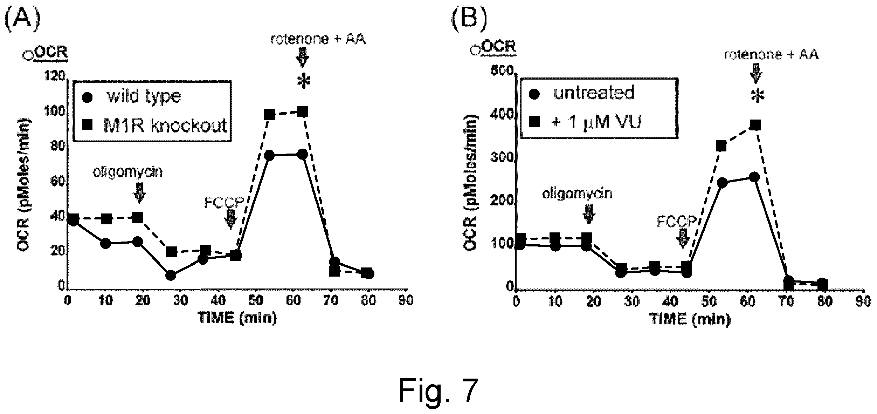

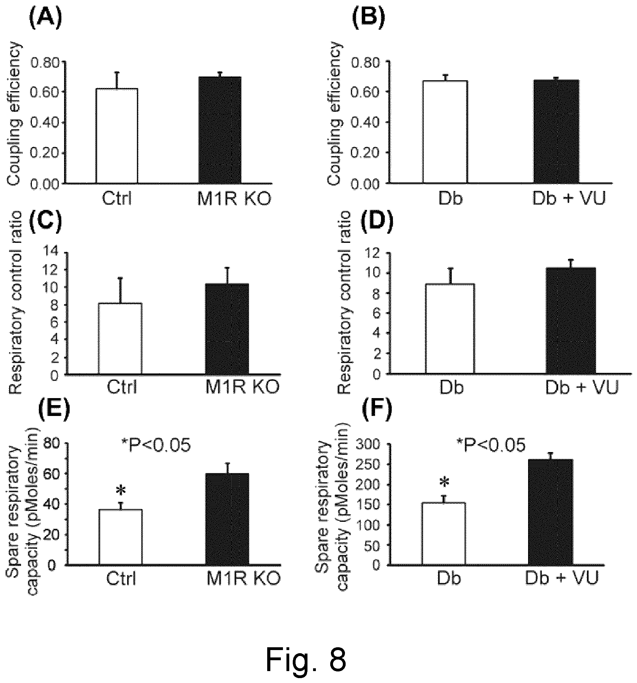

The data in Fig. 7(A) show that the oxygen consumption rate (OCR) was significantly augmented in neuron cultures derived from M1 receptor knockout mice (M1R KO) while the data in Fig. 7(B) show that treatment with VU0255035 resulted in increased OCR in neuron cultures derived from STZ-induced diabetic rats. The data in Figs. 8(A), 8(B) indicate that the coupling efficiencies in the neuron cultures from wild type mice, from M1R KO mice, and from diabetic mice that received dosing with VU0255035 were not dissimilar as also were their respiratory control rations (Figs. 8(C), 8(D)). However, the spare respirator capacity was significantly increased in neuron cultures from the M1R KO mice and from the VU0255035-treated cultures from diabetic rats compared to controls (Figs. 8(E), 8(F)) (the data shown in Figs. 8(A)-8(F) are means ± SEM of N=4-5 replicate cultures. Groups were compared using Student's t-test). This bioenergetics parameter, which is depressed in diabetes, is related to the capacity of cells to conduct respiration under conditions of stress or high ATP demand. In this regard we propose the stress is the presence of high intracellular glucose concentrations. Therefore, we propose that muscarinic receptor antagonism enhances AMPK/PGC-1α signaling to augment mitochondrial function that provides the energy in the form of ATP (from oxidative phosphorylation) for induction of axon regeneration.

### Example 6:

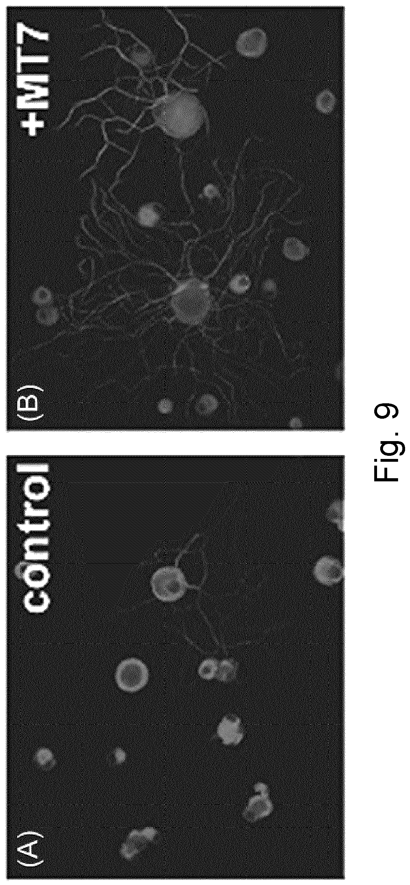

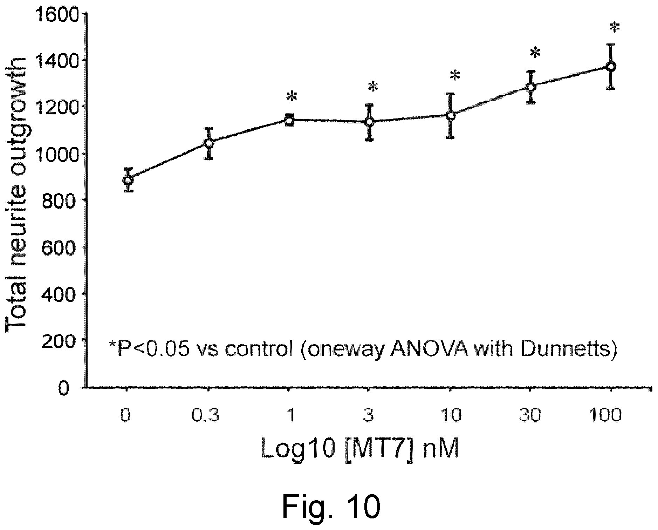

The highly specific M1 receptor antagonist, MT7 (muscarinic toxin 7), enhanced neurite outgrowth in cultured adult rat sensory neurons (Figs. 9(A), 9(B), 10). The data in Fig. 10 are means ± SEM of 9 replicate cultures. Significant differences were determined by one-way ANOVA with Dunnett's post-hoc test.

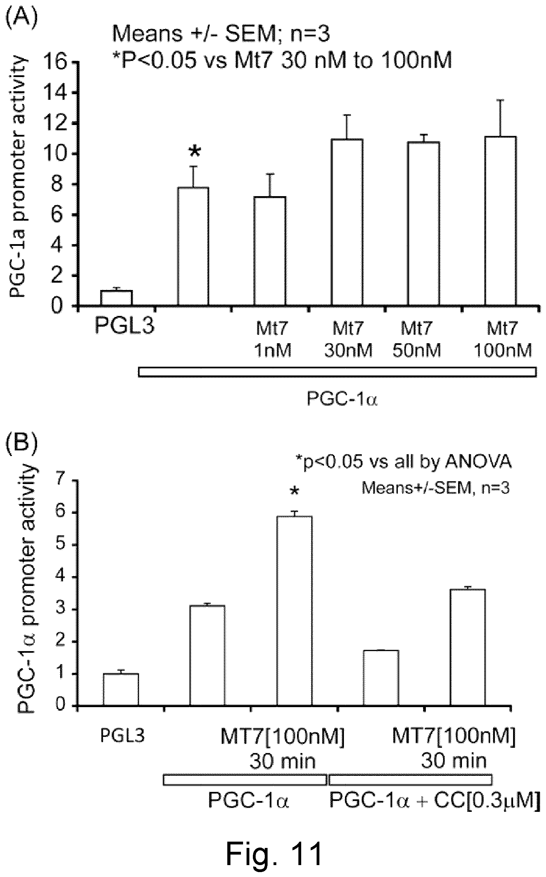

The data shown in Figs 11(A), 11(B) demonstrate that MT7 treatment activated transcription of PGC-la in cultured adult sensory neurons derived from STZ-induced diabetic rats, while co-treatment with 0.3µM of Compound C (CC), a pharmacological inhibitor of AMPK, blocked the stimulatory effects of MT7 (the data points in Figs. 11 are means ± SEM of N=3 replicate cultures. Significant differences were determined by oneway ANOVA with Tukey's post-hoc test). These data confirm that the AMPK/PGC-1α pathway is modulated by the M1 receptor, with specific blockade of the M1 receptor causing enhanced activity of AMPK/PGC-1α.

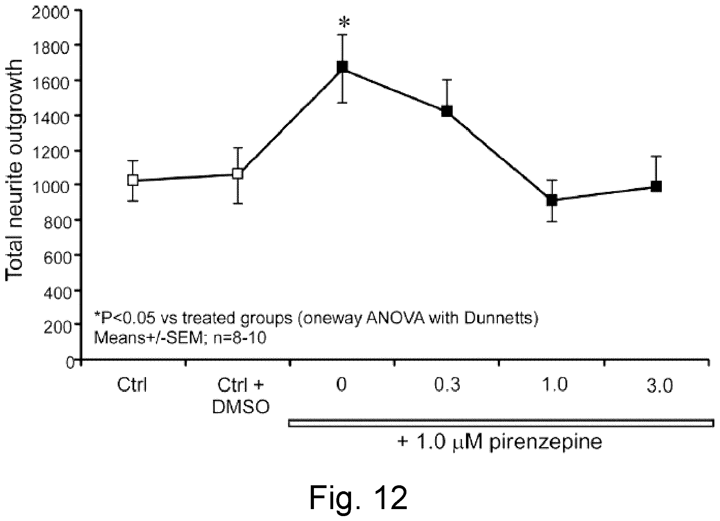

The data in Fig. 12 show that pirenzepine-induced neurite outgrowth in cultured adult sensory neurons derived from rats was dose-dependently inhibited by the CaMKK inhibitor, STO-609 (data are means ± SEM of N=8-10 replicate cultures. Significant differences were determined by oneway ANOVA followed by Dunnett's post-hoc test).

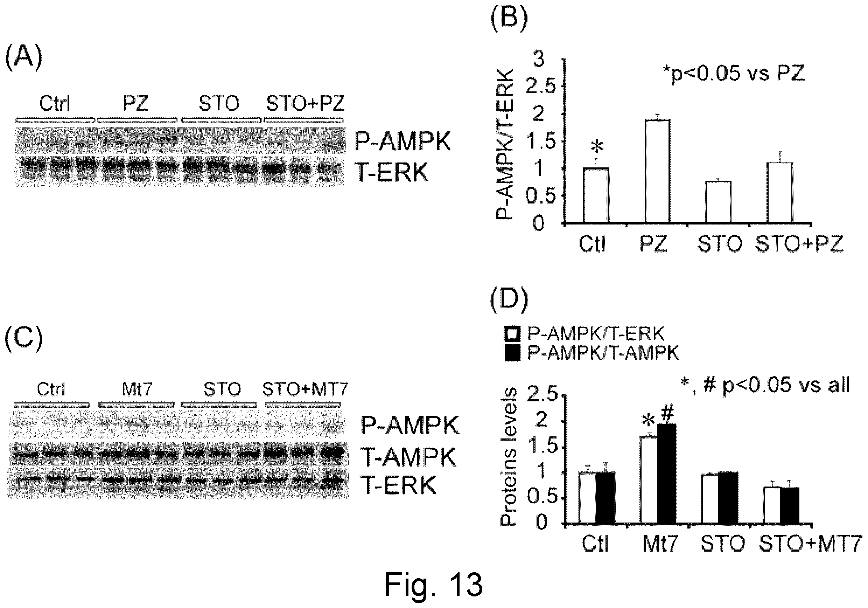

The data in Figs 13(A)-13(D) show that blockade of CaMKK using 1.0µM STO-609 inhibited the pirenzepine-induced or MT7-induced enhancement of AMPK phosphorylation in cultured adult sensory neurons derived from age-matched control rats (data are means ± SEM of N=3 replicate cultures. Significant differences were determined by oneway ANOVA followed by Dunnett's post-hoc test).

These data confirm that the induction of CaMKK, specifically CaMKKβ (or CaMKK2) is a proximal step in the pathway leading from blockade of the M1 receptor to activation of AMPK.

### Example 7:

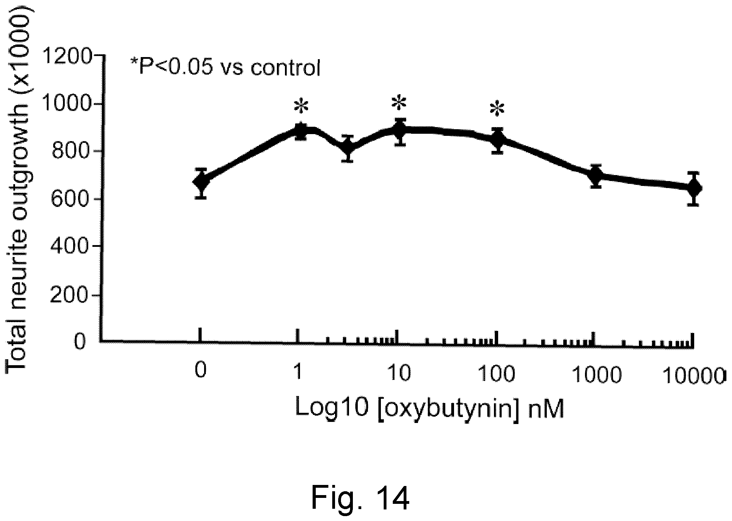

The data in Fig. 14 show that oxybutynin, a broad sprectrum M1, M2 and M3 receptor antagonist, elevated neurite outgrowth in cultured adult sensory neurons. This confirms that less selective antagonists of muscarinic receptors are also efficacious in enhancing axon regeneration *in vitro* (the data are means ± SEM of N=6 replicate cultures. Significant differences were determined with a oneway ANOVA followed by Dunnett's post-hoc test).

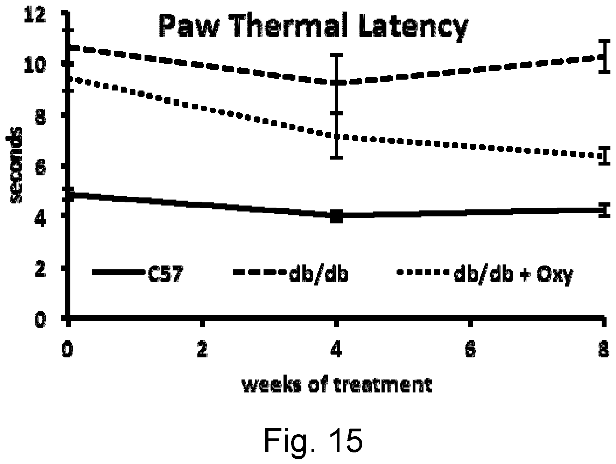

The muscarinic receptor antagonist oxybutynin was tested for efficacy against a functional measure of sensory neuropathy in the db/db mouse model of type 2 diabetes (Fig. 15). Thermal latency was analyzed as taught by Calcutt et al. (2004, Prevention of sensory disorders in diabetic Sprague-Dawley rats by aldose reductase inhibition or treatment with ciliary neurotrophic factor. Diabetologia 47:718-24). Female db/db mice were allowed to develop diabetes and small fiber sensory neuropathy was indicated by paw thermal hypoalgesia when compared to age-matched non-diabetic C57 mice (p<0.01). Some diabetic mice were then treated with oxybutynin (Oxy: 2% in hydrogel) applied to the right paw for 20 min/day 5 days a week, while others were treated with hydrogel vehicle alone. Treatment with oxybutynin for 8 weeks reversed paw thermal hypoalgesia in db/db mice so that values were significantly (p<0.01) lower than in vehicle treated mice, although they remained significantly (p<0.05) higher than in the non-diabetic C57 mice. Data are group mean ± SEM of N=8-9/group. At week 8, all other groups = p<0.01 vs db/db group by one-way ANOVA followed by Dunnett's post-hoc test.

These data show that thermal hypoalgesia was reversed in adult type 2 diabetic (db/db) mice treated with topical oxybutynin within 4-8 weeks of treatment. Thus this drug, which is currently used by humans to treat over-active bladder syndrome, was effective in reversing small fiber neuropathy in type 2 diabetes.

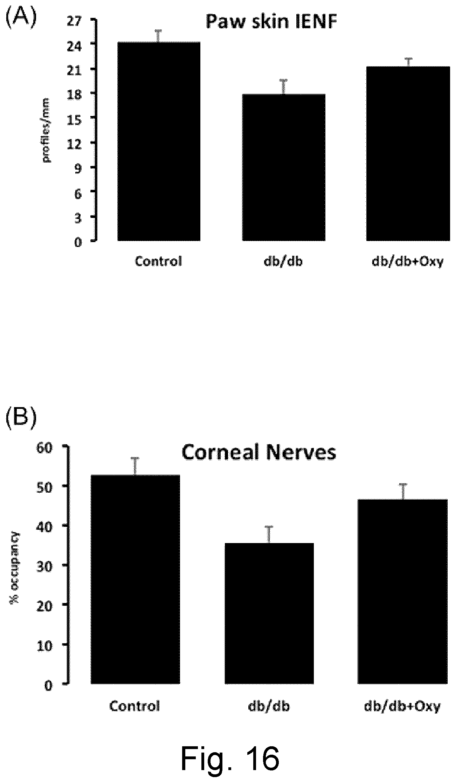

In this same study, treatment with oxybutynin was also effective in preventing loss of intraepidermal nerve fibers (IENF; Fig. 16(A)) and corneal nerves (Fig. 16(B)). IENF levels were quantified as taught by Beiswenger et al. (2008, Epidermal nerve fiber quantification in the assessment of diabetic neuropathy. Acta Histochem. 110:351-63). The data shown in Fig. 16(A) are group mean ± SEM of N=6-8/group. Significant differences were determined with a oneway ANOVA followed by Dunnett's post-hoc test. Vehicle-treated diabetic (db/db) mice showed significant (p<0.05) reductions in density of IENF in paw skin that was attenuated by oxybutynin treatment so that values were not significantly different from controls. The data shown in Fig. 16(B) show the effects of oxybutynin on levels of nerve fibers within the cornea of db/db diabetic mice calculated as the % occupancy of the cornea by nerve fibers. After 8 weeks of topical oxybutynin treatment, the eyes were assessed using corneal confocal microscopy as taught by Chen et al. (2013, Repeated monitoring of corneal nerves by confocal microscopy as an index of peripheral neuropathy in type-1 diabetic rodents and the effects of topical insulin. J. Periph. Nerv. Syst.18;306-315). Vehicle treated diabetic (db/db) mice showed a significant (p<0.05) reduction in nerve fiber occupancy in the cornea and thist was attenuated by oxybutynin treatment so that values were not significantly different from controls.

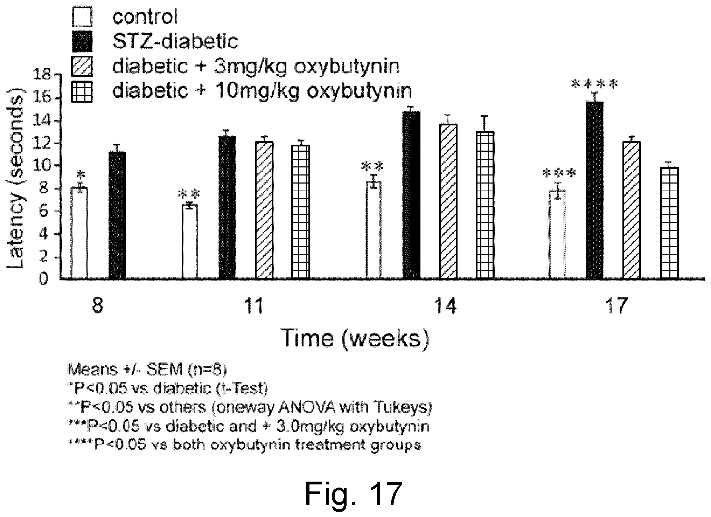

These data confirm that oxybutynin delivered via a topical route is able to reverse or prevent an array of small fiber deficits in type 2 diabetic mice. In Swiss Webster mice with type 1 diabetes that was induced by STZ, the systemic delivery of 3 mg/kg s.c. or 10 mg/kg s.c. oxybutynin for 9 weeks after an initial period of 8 weeks of untreated diabetes was also able to reverse thermal hypoalgesia (Fig. 17). Collectively, these data support the potential of selective M1R antagonists, including pirenzepine, VU0255035, MT7 and oxybutynin among others, to reverse established small fiber neuropathy in diabetes in addition to preventing functional indices of large fiber neuropathy (Fig. 2).

### Example 8:

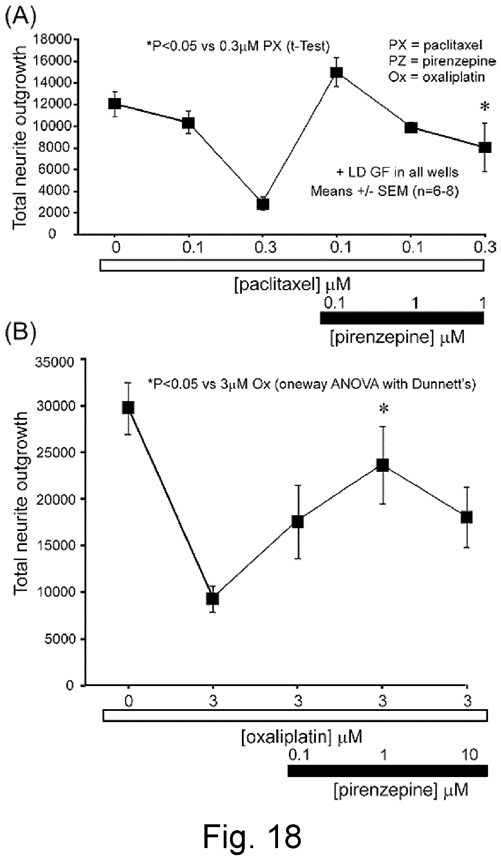

Given the phenotypic parallels between peripheral neuropathy induced by diabetes and chemotherapeutic agents, we have demonstrated that reduced neurite outgrowth of sensory neurons when exposed *in vitro* to paclitaxel (Fig. 18(A)) or to oxaliplatin (Fig. 18(B)) was prevented by pirenzepine. Adult sensory neurons from normal rats were cultured for 24 hours in the presence of (A) paclitaxel (PX; 0.1 µM or 0.3 µM), or (B) oxaliplatin (OX; 3.0 µM). From time of plating some cultures were treated with 0.1, 1.0 or 10 µM pirenzepine (PZ). Levels of total neurite outgrowth were determined and presented as means ± SEM, N=6-8/group. Significant differences were assessed in (A) by t-test, and in (B) by one-way ANOVA with Dunnett's post-hoc test.

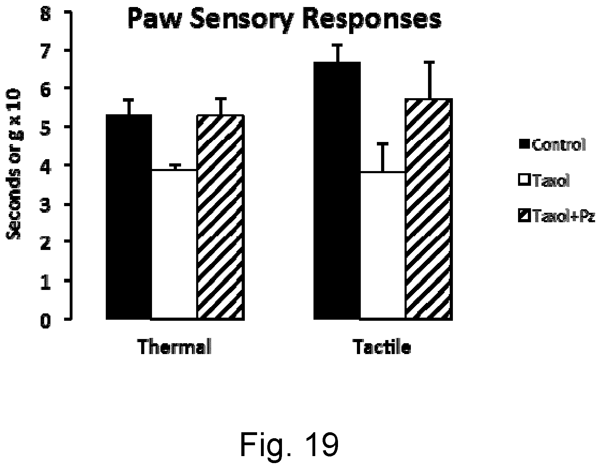

Swiss Webster mice were treated with paclitaxel (taxol; 5mg/kg on days 1,3,5 and 7) to induce peripheral neuropathy. Some paclitaxel-treated mice were also treated with pirenzepine (Pz: 10 mg/kg/day s.c.) for 4 weeks following paclitaxel treatment, while others were treated with vehicle alone. Fig. 19 shows paw thermal response latency (left panel) and paw 50% tactile response threshold (right panel) in mice treated with paclitaxel ± pirenzepine (PZ). Four weeks after paclitaxel treatment mice developed significant (p<0.01) paw thermal hyperalgesia and tactile allodynia, indicative of painful neuropathy transduced by small and large sensory fibers respectively. Data points are group mean of n=9-12/group ± SEM. Significant differences were determined with a oneway ANOVA followed by Dunnett's post-hoc test. The data shown in Fig. 19 demonstrate that paclitaxel (taxol)-treated mice developed peripheral neuropathy characterized by tactile allodynia and thermal hyperalgesia that was prevented by daily treatment with pirenzepine dosing at 1 mg/kg/day s.c.

### Example 9:

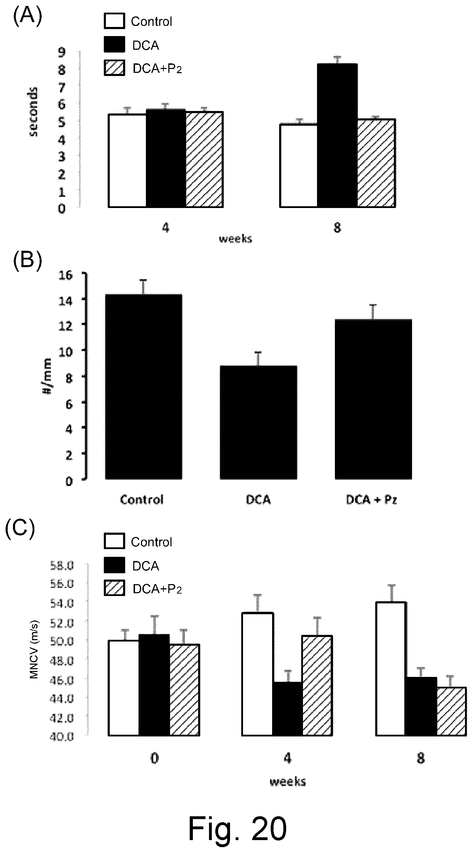

Another chemotherapeutic agent, dicholoroacetate (DCA), was also investigated and was shown to induce a peripheral neuropathy in mice that was characterized by sensory loss. Swiss Webster mice were treated with DCA (1mg/kg/day s.c.) for 8 weeks to induce peripheral neuropathy following the method described for rats by Calcutt et al. (2009, Peripheral neuropathy in rats exposed to dichloroacetate. J. Neuropath. Exp. Neurol. 68:985-93). Some DCA-treated mice were also treated with pirenzepine (Pz: 10 mg/kg/day s.c.) for the duration of the study while others were treated with vehicle alone. After 8 weeks of DCA treatment, mice developed significant (p<0.01) paw thermal hypoalgesia (Fig. 20(A)) indicative of small sensory fiber dysfunction, that was accompanied by significant (p<0.01) depletion of paw skin IENF (Fig. 20(B)). Both disorders were prevented by treatment with pirenzepine. Pirenzepine also delayed onset of MNCV slowing, an index of large fiber dysfunction, in DCA-treated mice after 4, but not 8, weeks of exposure (Fig. 20(C)). The data shown in Figs. 20(A)-20(C) represent group mean ± SEM with n=9-12/group. Significant differences were determined by oneway ANOVA followed by Dunnett's post-hoc test

Taken together, these data demonstrate that treatment with pirenzepine prevented indices of painful peripheral neuropathy as illustrated by thermal hyperalgesia and tactile allodynia, and also indices of degenerative neuropathy as illustrated by thermal hypoalgesia, IENF loss and MNCV slowing. Thus in two different models of chemotherapy-induced peripheral neuropathy (CIPN), systemic treatment with pirenzepine was able to protect from small and large fiber dysfunction and/or fiber loss.

### Example 10:

To extend our study of muscarinic receptor antagonists we treated STZ-induced diabetic mice with a broad spectrum drug, atropine. It is known that atropine blocks the activity of all muscarinic receptors.

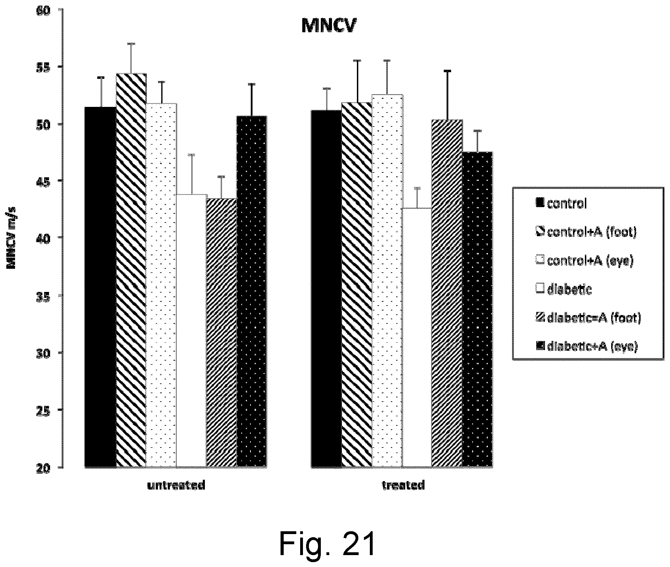

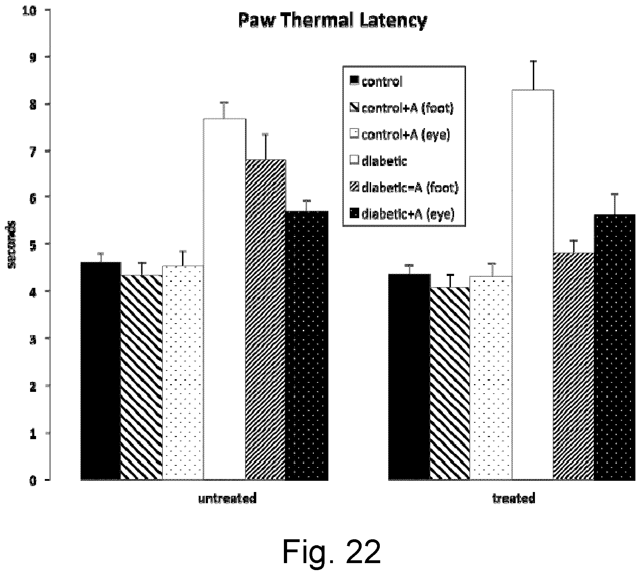

Groups of adult Swiss Webster mice were made diabetic with STZ and then treated 5 days/wk with atropine delivered topically to one eye (2% solution) or topically to one paw (2% gel) for 12 weeks. Measurements were made on both the treated and untreated hind paw. Indices of large motor fiber function (MNCV; Fig. 21) and sensory sensory fiber function (paw thermal latency; Fig. 22) were measured and compared to those of age-matched control mice that were treated in an equivalent manner. Diabetic mice showed significantly (p<0.01) reduced large fiber MNCV compared to control mice (Fig. 21), whereas diabetic mice treated with atropine delivered either to the eye or the foot had values not different to controls treated in the same manner (Fig. 21). Diabetic mice also showed significant (p<0.01) paw small sensory fiber-mediated thermal hypoalgesia compared to control mice (Fig. 22), whereas diabetic mice treated with atropine delivered either to the eye or the foot had values not different to controls treated in the same manner (Fig. 22). The data shown in Figs. 21 and 22 are group means ± SEM with N=6-10/group. Significant differences were determined by unpaired t-test to compare control and diabetic animals under each condition (vehicle, atropine to the eye, atropine to the foot).

These data confirm that delivery of atropine via the eye (by drops) or via the skin (topical application in hydrogel) caused protection from large and small fiber dysfunction in type 1 diabetic mice. Therefore selective M1 receptor antagonists, such as pirenzepine, VU0255035 and MT7, as well as broad-spectrum muscarinic receptor antagonists, such as oxybutynin and atropine, are efficacious in preventing and reversing peripheral neuropathy.

### Example 11:

To further extend our investigation of the actions of muscarinic receptor antagonists on nerve growth, we treated normal mice with the M1 receptor-specific antagonist MT7 by daily delivery to the eyes for 11 days and used confocal microscopy to provide a non-invasive and therefore iterative assessment of corneal nerve density.

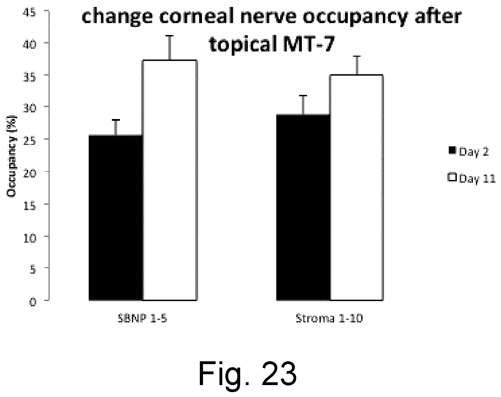

A group (n=8) of adult Swiss Webster mice received daily treatment with MT7, which was given by eye drops (20µl volume) in saline. Nerve occupancies in the sub-basal nerve plexus (SBNP 1-5) and upper stromal (Stroma 1-10) regions of the cornea were measured on day 2 and day 11 of treatment (Fig. 23). Nerve occupancy increased significantly (p<0.05 by paired test) in both regions by day 11 (white bars) when compared to values measured on day 2 (black bars). Data are group means (N=8) ± SEM.

These data demonstrate the efficacy of MT7 to promote growth of small sensory neurons of the cornea *in vivo.*

### Example 12:

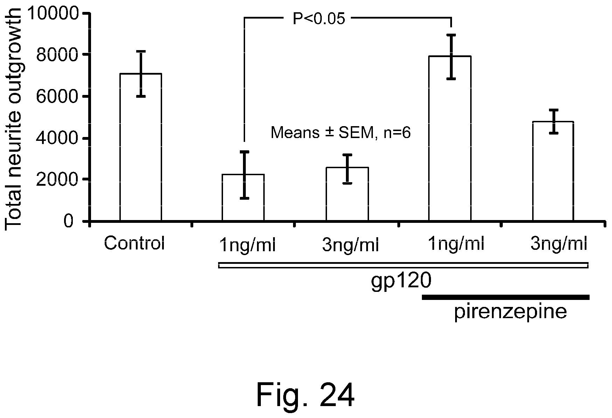

To further extend our investigation of the actions of muscarinic receptor antagonists on nerve growth, cultured adult rat sensory neurons were treated for 24 hours with recombinant gp120, an external coat protein of HIV that causes toxicity to sensory neurons in HIV neuropathy (Fig. 24). This toxicity leads to axon degeneration and neuronal cell death. Cultures were treated with 1µM pirenzpeine and total neurite outgrowth determined. Pirenzepine treatment afforded complete protection from gp120-induced neurite degeneration (P<0.05 by oneway ANOVA with Tukeys post-hoc test). MT7 also exhibited approximately 50% level of protection from gp120 treatment (not shown). Values are means ± SEM, n=6.

## Claims

1. **A composition for use in a method of treating a peripheral neuropathy disorder, the composition comprising an effective amount of pirenzepine or salt thereof and a pharmacologically** acceptable carrier and/or an excipient; wherein the peripheral neuropathy is one of a chemotherapy-induced peripheral neuropathy, a compression-induced peripheral neuropathy, a peripheral neuropathy induced by an exposure to toxic agent, cancer or tumor induced neuropathy, an immune-mediated peripheral neuropathy, a peripheral neuropathy induced by an infection or infectious disease, a genetically acquired peripheral neuropathy, a idiopathic neuropathy, a surgically-induced neuropathy, polyneuropathy, mononeuropathy, **or an acute pain autonomic neuropathy.**

2. The composition for use as claimed in claim 1, wherein pirenzepine comprises from 0.1% to 10% by weight of composition.

3. The composition for use as claimed in claim 1, wherein the composition is injectable.

4. The composition for use as claimed in claim 1, wherein the composition is administrable by a topical application.

5. The composition for use as claimed in claim 4, wherein the composition is one of a lotion, a cream, a gel, and a viscous fluid.

6. The composition for use as claimed in claim 4, additionally comprising one or more of a penetration enhancer, an emollient, an emulsifying agent, a water miscible solvent, an alcohol, and mixtures thereof.

7. The composition for use as claimed in claim 6, wherein the penetration enhancer is about 0.5% by weight of the composition.

8. The composition for use as claimed in claim 4, wherein the composition is in the form of a transdermal patch.

9. The composition for use as claimed in claim 1, wherein the composition is administrable by an oral dosage.

10. The composition for use as claimed in any of claims 1-9, wherein the peripheral neuropathy disorder comprises a chemotherapy-induced peripheral neuropathy.

11. The composition for use as claimed in any of claims 1-9, wherein the peripheral neuropathy disorder comprises a peripheral neuropathy induced by an infectious disease.

12. The composition for use as claimed in any of claim 11, wherein the infectious disease comprises HIV.

## Patentansprüche

1. Zusammensetzung zur Verwendung in einem Verfahren zur Behandlung einer peripheren Nervenkrankheit, wobei die Zusammensetzung eine wirksame Menge Pirenzepin oder eines Salzes davon und einen pharmakologisch akzeptablen Träger und/oder Hilfsstoff umfasst; wobei die periphere Nervenkrankheit eine der folgenden ist: eine durch Chemotherapie induzierte periphere Neuropathie, eine durch Kompression induzierte periphere Neuropathie, eine durch eine Exposition einer toxischen Substanz induzierte periphere Neuropathie, eine durch Krebs oder Tumor induzierte Neuropathie, eine immunvermittelte periphere Neuropathie, eine durch eine Infektion oder eine Infektionskrankheit induzierte periphere Neuropathie, eine genetisch erworbene periphere Neuropathie, eine idiopathische Neuropathie, eine chirurgisch induzierte Neuropathie, Polyneuropathie, Mononeuropathie oder eine autonome Neuropathie mit akuten Schmerzen.

2. Zusammensetzung zur Verwendung nach Anspruch 1, wobei Pirenzepin 0,1 Gew.-% bis 10 Gew.-% der Zusammensetzung umfasst.

3. Zusammensetzung zur Verwendung nach Anspruch 1, wobei die Zusammensetzung injizierbar ist.

4. Zusammensetzung zur Verwendung nach Anspruch 1, wobei die Zusammensetzung durch topische Anwendung verabreicht werden kann.

5. Zusammensetzung zur Verwendung nach Anspruch 4, wobei die Zusammensetzung eines der folgenden ist: eine Lotion, eine Creme, ein Gel oder ein viskoses Fluid.

6. Zusammensetzung zur Verwendung nach Anspruch 4, wobei diese zusätzlich eines oder mehrere der folgenden umfasst: einen Penetrationsverstärker, einen Weichmacher, einen Emulgator, ein wassermischbares Lösungsmittel, einen Alkohol und Gemische davon.

7. Zusammensetzung zur Verwendung nach Anspruch 6, wobei der Penetrationsverstärker etwa 0,5 Gew.-% der Zusammensetzung entspricht.

8. Zusammensetzung zur Verwendung nach Anspruch 4, wobei die Zusammensetzung in Form eines transdermalen Patches gegeben ist.

9. Zusammensetzung zur Verwendung nach Anspruch 1, wobei die Zusammensetzung über eine orale Dosis verabreicht werden kann.

10. Zusammensetzung zur Verwendung nach einem der Ansprüche 1 bis 9, wobei die periphere Nervenkrankheit eine durch Chemotherapie induzierte periphere Neuropathie umfasst.

11. Zusammensetzung zur Verwendung nach einem der Ansprüche 1 bis 9, wobei die periphere Nervenkrankheit eine durch eine Infektionskrankheit induzierte periphere Neuropathie umfasst.

12. Zusammensetzung zur Verwendung nach Anspruch 11, wobei die Infektionskrankheit HIV umfasst.

## Revendications

1. Composition destinée à être utilisée dans un procédé de traitement d'un trouble de neuropathie périphérique, la composition comprenant une quantité efficace de pirenzépine ou d'un sel associé et un vecteur et/ou un excipient pharmacologiquement acceptable ; la neuropathie périphérique étant une neuropathie périphérique induite par la chimiothérapie, une neuropathie périphérique induite par compression, une neuropathie périphérique induite par une exposition à un agent toxique, une neuropathie induite par un cancer ou une tumeur, une neuropathie périphérique à médiation immunitaire, une neuropathie périphérique induite par une infection ou une maladie infectieuse, une neuropathie périphérique acquise génétiquement, une neuropathie idiopathique, une neuropathie induite chirurgicalement, une polyneuropathie, une mononeuropathie ou une neuropathie autonome de douleur aiguë.

2. Composition destinée à être utilisée selon la revendication 1, la pirenzépine constituant de 0,1 % à 10 % en poids de la composition.

3. Composition destinée à être utilisée selon la revendication 1, la composition étant injectable.

4. Composition destinée à être utilisée selon la revendication 1, la composition pouvant être administrée par une application topique.

5. Composition destinée à être utilisée selon la revendication 4, la composition étant une lotion, une crème, un gel ou un fluide visqueux.

6. Composition destinée à être utilisée selon la revendication 4, comprenant en outre un activateur de pénétration, un émollient, un agent émulsifiant, un solvant miscible à l'eau, un alcool, et/ou leurs mélanges.

7. Composition destinée à être utilisée selon la revendication 6, l'activateur de pénétration représentant environ 0,5 % en poids de la composition.

8. Composition destinée à être utilisée selon la revendication 4, la composition étant sous la forme d'un patch transdermique.

9. Composition destinée à être utilisée selon la revendication 1, la composition pouvant être administrée par un dosage oral.

10. Composition destinée à être utilisée selon l'une quelconque des revendications 1 à 9, le trouble de neuropathie périphérique comprenant une neuropathie périphérique induite par la chimiothérapie.

11. Composition destinée à être utilisée selon l'une quelconque des revendications 1 à 9, le trouble de neuropathie périphérique comprenant une neuropathie périphérique induite par une maladie infectieuse.

12. Composition destinée à être utilisée selon la revendication 11, la maladie infectieuse comprenant le VIH.
